# EUROPÄISCHE PATENTSCHRIFT

(11) **EP 3 066 596 B1**
(45) Veröffentlichungstag und Bekanntmachung des Hinweises auf die Patenterteilung: **11.05.2022**
(21) Anmeldenummer: 14707701.0
(22) Anmeldetag: 20.02.2014
(51) Int. Cl.: G16H 30/20

(54) **SYSTEM ZUR ANZEIGE UND BEARBEITUNG VON DATEN EINER MEDIZINISCHEN EINRICHTUNG**
SYSTEM FOR DISPLAYING AND EDITING DATA FOR A MEDICAL DEVICE
SYSTÈME DE VISUALISATION ET DE TRAITEMENT DE DONNÉES D'UN DISPOSITIF MÉDICAL

(43) Veröffentlichungstag der Anmeldung: 14.09.2016
(73) Patentinhaber: Siemens Healthcare GmbH, 91052 Erlangen (DE)
(72) Erfinder: DORN, Karlheinz, 90562 Kalchreuth (DE)
(86) Internationale Anmeldenummer: PCT/EP2014/053356
(87) Internationale Veröffentlichungsnummer: WO 2015/124192

(56) Entgegenhaltungen:
- US-A1- 2005 228 250
- US-A1- 2010 049 740
- US-A1- 2012 185 874
- US-A1- 2013 185 318
- US-A1- 2013 208 966
- US-A1- 2013 290 404
- US-A1- 2013 308 839
- US-B1- 7 702 719
- US-B2- 9 953 136
- BAHGA ARSHDEEP ET AL: "A Cloud-based Approach for Interoperable Electronic Health Records (EHRs)", IEEE JOURNAL OF BIOMEDICAL AND HEALTH INFORMATICS, IEEE, PISCATAWAY, NJ, USA, Bd. 17, Nr. 5, 1. September 2013 (2013-09-01), Seiten 894-906, XP011525485, ISSN: 2168-2194, DOI: 10.1109/JBHI.2013.2257818 [gefunden am 2013-08-30]
- PATEL G: "DICOM Medical Image Management the challenges and solutions: Cloud as a Service (CaaS)", COMPUTING COMMUNICATION&NETWORKING TECHNOLOGIES (ICCCNT), 2012 THIRD INTERNATIONAL CONFERENCE ON, IEEE, 26. Juli 2012 (2012-07-26), Seiten 1-5, XP032443043, DOI: 10.1109/ICCCNT.2012.6396083

## Beschreibung

Die Erfindung bezieht sich auf ein System zur Verwaltung und Bearbeitung von Daten einer medizinischen Einrichtung.

Unter dem Begriff "Verwaltung" wird hier und im Folgenden insbesondere die Archivierung der Daten (das heißt die Einstellung der Daten in einen persistenten Datenspeicher), die Wiedergabe (Anzeige) und das Löschen der Daten aus dem Datenspeicher sowie das Sortieren und Auffinden bestimmter Daten aus dem Datenspeicher nach Maßgabe vorgegebener Kriterien (Browsing) verstanden.

Unter dem Begriff "Bearbeitung" wird hier und im Folgenden insbesondere die Änderung (Editierung / Aufbereitung) der Daten verstanden.

Die zu verwaltenden und zu bearbeitenden Daten der medizinischen Einrichtung umfassen insbesondere Patientendaten, Aufgaben (Works/Tasks) oder Aufgabenlisten (Worklists) für das Personal der medizinischen Einrichtung und medizinische Bilddaten.

Die Verwaltung solcher medizinischer Daten erfolgt in zunehmendem Maße computergestützt durch Server-Systeme, insbesondere sogenannte Informationssysteme. Ein Informationssystem umfasst regelmäßig
- einen oder mehrere Datenspeicher, beispielsweise in Form eines Storage Area Network (SAN),
- einen oder mehrere zugehörige Datenserver,
- mindestens eine relationale Datenbank, die in einem Datenbankserver implementiert ist,
- sowie in der Regel einen oder mehrere weitere Server, in denen Methoden für den Datenbankzugriff und die Datenbearbeitung implementiert sind.

Für die unterschiedlichen Datenarten haben sich im medizinischen Bereich unterschiedliche Informationssysteme etabliert. So werden im Umfeld einer medizinischen Einrichtung wie zum Beispiel einer Klinik regelmäßig ein "Krankenhaus-Informations-System" (Hospital Information System, HIS) für die Verwaltung und Bearbeitung der Patientendaten und ein "Radiologie-Informations-System" (RIS) für die Terminplanung radiologischer Untersuchungen, die Unterstützung der Befundung medizinischer Bilddaten und die Dokumentation der Befunde eingesetzt. Daneben umfasst die IT-Struktur eines Krankenhauses in der Regel ein sogenanntes "Picture Archiving and Communication System" (PACS) zur Archivierung und Übertragung von medizinischen Bilddaten auf Basis des DICOM-Standards sowie ein "Avanced Visualisation(AV)-System", das servergestützte Funktionen zur Visualisierung von Volumendaten, insbesondere dynamisches Volume Rendering, zur Verfügung stellt.

Die vorstehend bezeichneten Server-Systeme sind dabei in der Regel parallel zueinander vorhanden. Dies erfordert einen hohen Beschaffungs- und Wartungsaufwand, der insbesondere für kleine medizinische Einrichtungen oder sonstige Einrichtung mit vergleichsweise geringem Finanzvolumen kaum zu bewältigen ist.

Die vorstehend beschriebene, komplexe IT-Struktur einer modernen medizinischen Einrichtung weist zudem nur vergleichsweise schlechte Skalierungseigenschaften auf. Eine Anpassung einer solchen IT-Struktur an größere Änderungen des zu verarbeitenden und zu archivierenden Datenvolumens und/oder der erforderlichen Rechenleistung ist somit meist nur mit sehr hohem Aufwand möglich.

Als Nutzer- oder Endgeräte (klassischerweise als Clients bezeichnet) einer solchen IT-Struktur werden bisher überwiegend Personal Computer (PCs) verwendet, wobei diese PCs oft als sogenannte "Thin Clients" ausgebildet sind, die einen Großteil der erforderlichen Rechenleistung von einem angeschlossenen Server beziehen. In jüngerer Zeit wird allerdings zunehmend gewünscht, auch mobile Kleinrechner wie Smartphones, Tablets oder PDAs als Nutzergerät zu verwenden.

Ein weiteres Problem herkömmlicher Informations-Systeme im medizinischen Umfeld besteht darin, dass die Frontend-Software dieser Systeme oft spezifisch und starr auf die Verwaltung und Bearbeitung bestimmter Datenarten ausgerichtet ist. Dies führt dazu, dass das Frontend für jedes Informationssystem eigens programmiert und gepflegt werden muss. Dies wiederum erschwert insbesondere die Integration neuartiger Nutzergeräte wie Smartphones und Tablets in den klinischen Arbeitsablauf, da die mit der entsprechenden Anpassung der jeweiligen Frontends verbundene Diversifizierung der Softwarekomponenten vom Herstellungs- und Weiterentwicklungsaufwand her nur mit großem Aufwand handhabbar ist. Dies betrifft insbesondere Software zum Anzeigen (allgemein als "Viewing" bezeichnet) von Bilddaten, da entsprechende Applikationen für verschiedene Bilddatenarten sowie für verschiedene Einsatzzwecke, z.B. vorläufige Durchsehen der Bilddaten an der bilderzeugenden Modalität (Examination), das eigentliche Befunden (Reading) sowie ggf. das bloße Ansehen bereitgestellt werden müssen.

Als Alternative zu herkömmlichen Client-Server-Architekturen haben sich in den vergangenen Jahren zunehmend sogenannte Cloud-Lösungen etabliert. Als "Cloud" ("Rechnerwolke") wird dabei eine Datenverarbeitungseinrichtung verstanden, die von einem von dem Nutzer unabhängigen Cloud-Betreiber ("Cloud Vendor") zur Verfügung gestellt und betrieben wird. Der "Cloud Vendor" stellt hierbei einer Vielzahl von Nutzern die Hardware und gegebenenfalls die Software der Cloud im Rahmen eines Nutzungsvertrags (Subscription) als Dienst zur Verfügung. Je nach dem Umfang der zur Verfügung gestellten Dienste unterscheidet man zwischen
- einem als "Infrastructure as a Service" (IaaS) bezeichneten Nutzungsmuster, bei dem dem Nutzer lediglich Computerhardware (Rechner, Netzwerke und Speicher) der Cloud zur Verfügung gestellt wird, während der Nutzer für die in der Cloud betriebene Software in vollem Umfang selbst verantwortlich ist,
- einem als "Platform as a Service" (PaaS) beschriebenen Nutzungsmuster, bei dem dem Nutzer aus der Cloud die Computerhardware zusammen mit einer darauf aufbauenden Programmierungs- und Laufzeitumgebung angeboten wird, so dass der Nutzer nur für die in dieser Programmierungsund Laufzeitumgebung implementierte Anwendungssoftware (Applikationen) selbst verantwortlich ist, sowie
- einem als "Software as a Service" (SaaS) bezeichneten Nutzungsmuster, bei dem dem Nutzer darüber hinaus auch bestimmte Anwendungssoftware aus der Cloud zur Verfügung gestellt wird.

Die US 7 702 719 B1 offenbart ein Verfahren zur Verwendung in einem Client / Server-System zum Reduzieren von Interaktionen zwischen einem Client und einem Server. Dabei wird der Server zum Speichern eines der Anwendung zugeordneten Modells und Ausführen einer mit der Anwendung verbundenen Ansichtserzeugungs- und Steuerlogik konfiguriert. Der Client wird dahingehend konfiguriert, dass er mindestens eine Teilmenge des der Anwendung zugeordneten Modells speichert und mindestens eine Teilmenge der der Anwendung zugeordneten Ansichtserzeugungsund Steuerlogik ausführt, wobei ein oder mehrere Teile der Anwendung auf dem Client ausgeführt werden, ohne dass der Client mit dem Server interagieren muss, und wobei der Client und der Server beide lokal mindestens einen Teil des Modells verwalten und die damit verbundene Ansichtserzeugungs- und Steuerlogik ausführen.

Die US 2013 / 208 966 A1 offenbart eine Ausführungsform, bei der ein lokales Gerät medizinische 3D-Bilddaten empfängt, die von einem medizinischen Bildgebungsgerät erfasst wurden. Die Bilddaten werden anonymisiert, indem bestimmte mit den Bilddaten verknüpfte Metadaten basierend auf einer Anonymisierungsvorlage entfernt werden. Das lokale Gerät lädt die anonymisierten Bilddaten automatisch über ein Netzwerk auf einen Cloud-Server hoch, und zwar basierend auf einer oder mehreren Regeln. Dabei wird eine Netzwerkverbindung verwendet, die über einen Internet-Port des lokalen Geräts hergestellt wird. Der Cloud-Server ist so konfiguriert, dass er einer Vielzahl von Benutzern medizinische Bildverarbeitungsdienste unter Verwendung einer Vielzahl von durch den Cloud-Server bereitgestellten Bildverarbeitungswerkzeugen bereitstellt.

Die US 2013 / 308 839 A1 offenbart integrierte medizinische Softwaresysteme, bei denen mindestens ein Teil der medizinischen Informationen eines Patienten in einem MRCS angezeigt wird, das in einem lokalen Gerät ausgeführt wird, wobei die medizinischen Informationen die Krankengeschichte des Patienten enthalten. Mindestens ein Teil der angezeigten medizinischen Informationen des Patienten wird über ein Netzwerk an einen medizinischen Bildverarbeitungsserver übertragen, wobei die übertragenen medizinischen Informationen eine Patientenkennung (ID) des Patienten enthalten. Sowohl der mindestens eine Teil der medizinischen Patienteninformationen als auch eines oder mehrere medizinische Bilder werden in dem MRCS angezeigt, wobei die medizinischen Bilder dem Patienten zugeordnet sind und von dem medizinischen Bildverarbeitungsserver wiedergegeben werden.

Je nach dem Nutzerkreis, an den die jeweilige Cloud adressiert ist, unterscheidet man des Weiteren zwischen
- einer sogenannten Public Cloud, deren Dienste von jedem in Anspruch genommen werden können, und
- einer sogenannten Privat Cloud, die nur Nutzern einer bestimmten Organisation, insbesondere eines bestimmten Konzerns zugänglich ist.

Für jeden Nutzer einer Public Cloud sind die Zugriffsberechtigungen auf bestimmte Hardware- und Softwarebestandteile der Cloud durch die dem Nutzer zugeordnete Subscription geregelt. Public Clouds sind hierdurch regelmäßig "mandantenfähig" (multi-tenant). Dies bezeichnet die Fähigkeit, Daten, Benutzerverwaltung und Rechenoperationen für Nutzer mit verschiedener Subscription strikt getrennt zu halten. Ein Nutzer der Public Cloud kann also in die Daten, Benutzerverwaltung und Rechenoperationen eines anderen Nutzers mit unterschiedlicher Subscription keinen Einblick nehmen und diese Daten auch nicht beeinflussen.

Allerdings werden durch eine bloße Übertragung der klassischerweise gebräuchlichen Server-Systeme in die Cloud die eingangs beschriebenen Probleme nicht gelöst, zumal hierdurch die Komplexität der IT-Struktur nicht vereinfacht, sondern eher sogar noch erhöht wird. Andererseits entstehen hierdurch neue Probleme. Insbesondere kann es bei einer Verlagerung der IT-Struktur in die Cloud zu einer spürbaren Verlängerung der Latenzzeiten für die Bearbeitung von Nutzeranfragen kommen. Dies ist insbesondere lästig bei Viewing-Applikationen zur Anzeige von medizinischen Bilddaten, da latenzzeitbedingt der Aufbau der zu betrachtenden Bilder dem Arbeitstempo eines Benutzers spürbar nachhinken kann, was zu einer merklichen Beeinträchtigung der Arbeitsabläufe im medizinischen Umfeld führen kann.

Der Erfindung liegt die Aufgabe zugrunde, ein System zur Verwaltung und Bearbeitung von Daten einer medizinischen Einrichtung anzugeben, das besonders flexibel einsetzbar ist.

Diese Aufgabe wird erfindungsgemäß gelöst durch die Merkmale des Anspruchs 1. Vorteilhafte und teils für sich gesehen erfinderische Ausgestaltungen und Weiterentwicklungen der Erfindung sind in den Unteransprüchen und der nachfolgenden Beschreibung dargelegt.

Das erfindungsgemäße System dient insbesondere zur Anzeige und Bearbeitung von medizinischen Bilddatensätzen einer oder mehrerer der folgenden Bilddatenarten:
- 2D-Bilddaten, insbesondere zweidimensionale, unbewegte, medizinische Einzelbilder wie z.B. Röntgen- und Ultraschallbilder, sogenannte Keyimages, Photos und Dokumente, aber auch 2D-Bildsequenzen oder Videos,
- 3D-Bilddaten (auch als Volumendaten bezeichnet) wie z.B. Computertomogramme und MR-Tomogramme und/oder
- zeitliche Volumendatensequenzen (4D-Bilddaten).

Als "Keyimage" wird hierbei ein Schnappschuss, d.h. ein (bearbeiteter oder unbearbeiteter) Ausschnitt aus einem medizinischen Bild- oder Volumendatensatz bezeichnet, der bei der Befundung dieser Daten zur Darstellung eines medizinischen Befundes abgeleitet wird. Solche Keyimages werden typischerweise als Bestandteil eines medizinischen Berichts (Report) verwendet.

Als "Photos" werden - unter Abgrenzung von den zuvor genannten medizinischen Einzelbildern und den Keyimages - ein Bild bezeichnet, dass im medizinischen Kontext nur mittelbare Relevanz hat, z.B. Portraitbilder von Patienten oder medizinischem Personal, Bilder von medizinischen Einrichtungen, photographische Kopien von Texten, etc.

Als "Dokumente" werden Dateien mit Bild- und/oder Textinhalt bezeichnet, die nicht in einem originären Bilddatenformat, sondern in einem Dokumentenformat (z.B. PDF) vorliegen.

Eine 2D-Bildsequenz umfasst mehrere zweidimensionale Einzelbilder. Diese Einzelbilder können - ähnlich wie bei einem Video - die zeitliche Änderung einer Bildinformation wiedergeben. Allerdings können auch Einzelbilder, die nicht in einem unmittelbaren zeitlichen Zusammenhang stehen, in einer 2D-Bildsequenz zusammengefasst werden, z.B. verschiedene Projektionen eines Körperbereichs eines Patienten.

Zu unterscheiden sind die vorstehend aufgeführten Bilddaten dabei von anderen medizinischen Daten, die optional ebenfalls durch das System verwaltet und bearbeitet werden, z.B.:
- Patientendaten, die persönliche und medizinische Angaben zu einem bestimmten Patienten der Einrichtung umfassen, insbesondere Angaben zum Namen, der Adresse, dem Alter, dem Geschlecht, früheren Diagnosen und Behandlungen,
- Aufgaben (Works/Tasks) und Aufgabenlisten (Worklists), die von einem oder mehreren medizinischen Nutzern (Ärzten, medizinischen Assistenzkräften, Pflegern etc.) der medizinischen Einrichtung durchzuführen sind, sowie
- Kontextdaten, das heißt Kontextinformation zu medizinischen Bilddaten, insbesondere Verweise auf den Speicherort von Bilddatensätzen sowie optional Meta-Daten zu Bilddatensätzen wie z.B. die der jeweiligen Bildaufnahme zugrundeliegenden Aufnahmeparameter.

Die Datensätze einer bestimmten Datenart sind nachfolgend auch als "Ressource" bezeichnet.

Zur Anzeige von Bilddaten mindestens einer bestimmten Bilddatenart umfasst das System mindestens eine Viewing-Applikation zum Ablauf auf einem Nutzergerät.

Erfindungsgemäß ist die Viewing-Applikation hinsichtlich ihrer Komponenten-Architektur in zumindest vier Schichten gegliedert. Diese Schichten umfassen eine View-Schicht, eine ViewModel-Schicht, eine Model-Schicht und eine Treiberschicht.

Die View-Schicht umfasst eine Anzahl von Komponenten (also mindestens eine Komponente, optional aber auch mehrere Komponenten), die dazu eingerichtet sind, den graphischen Gehalt einer GUI-Seite zur Anzeige von 2D-Bilddaten oder zur Anzeige von 3D-Bilddaten zu definieren. In einer bevorzugten Ausführung umfasst die View-Schicht hierbei zwei Komponenten (beispielhaft als "ReadingView2D" und "ReadingView3D" bezeichnet), von denen die erste ein ausschließlich zur Darstellung von 2D-Bilddaten (einschließlich Keyimages, Photos, 2D-Bildsequenzen und Videos) eingerichtetes GUI definiert, während die zweite ein ausschließlich zur Darstellung von 3D-Bilddaten eingerichtetes GUI definiert. Die letztgenannte Komponente gehört dabei vorzugweise nicht zu der Basisausstattung der Viewing-Applikation, sondern ist optional auf Nutzeranforderung oder im Rahmen einer Lizenzvereinbarung zuladbar oder aktivierbar.

Die oder jede Komponente der View-Schicht definiert hierbei insbesondere die Art, das Aussehen und Anordnung der graphischen Elemente der zugehörigen GUI-Seite, insbesondere der Steuerelemente, über die Information an einen Nutzer ausgegeben oder Nutzereingaben und -befehle aufgenommen werden können. Durch die Komponenten der View-Schicht wird dagegen nicht die mit den Steuerelementen verbundene Logik (UI-Logik) festgelegt, da dies der ViewModel-Schicht vorbehalten ist. Als GUI-Seite wird hierbei ein abgeschlossener und für sich gesehen funktionsfähiger Teil einer graphischen Nutzeroberfläche (GUI) bezeichnet, der - etwa nach Art eines Fensters - alleine oder im Verbund mit weiteren GUI-Seiten betrieben werden kann.

Die ViewModel-Schicht umfasst eine (in zweckmäßiger Ausführung lediglich eine einzige) Komponente, die beispielhaft als "ReadingViewModel" bezeichnet ist. Wie vorstehend angedeutet, implementiert die Komponente der ViewModel-Schicht die UI-Logik, also die Eigenschaften und Befehle für die Steuerelemente der in der oder jeder Komponente der View-Schicht definierten GUI-Seite. Das "ReadingViewModel" arbeitet vorzugsweise asynchron und parallel und stellt einen Mediator zwischen der View-Schicht und der Treiberschicht dar.

Die Model-Schicht umfasst eine (in zweckmäßiger Ausführung lediglich eine einzige) Komponente, die beispielhaft als "<Tweet>ReadingModel" bezeichnet ist. Diese Komponente definiert hierbei Variablen für die anzuzeigenden Bilddaten. Sie hält somit im Betrieb der Applikation die anzuzeigenden Bilddaten.

Die Treiberschicht umfasst zumindest eine Treiberkomponente (nachfolgend als "ReadingService2D" bezeichnet), die dazu eingerichtet ist, 2D-Bilddatensätze aus einem Bilddatenspeicher zu laden und für die Anzeige auf einem Bildschirm aufzubereiten. Im Falle von 2D-Bilddaten besteht die Aufbereitung hierbei insbesondere in der Drehung, der Verschiebung und/oder der Vergrößerung/Verkleinerung der Bildinformation oder - bei Bilddaten, deren Bildinformation in eine Vielzahl von Bildkacheln unterteilt ist - in der Auswahl der richtigen Bildkachel.

Die Treiberschicht umfasst des Weiteren eine Treiberkomponente (nachfolgend als "ReadingServiceAgent3D" bezeichnet), die dazu eingerichtet ist, 3D-Bilddatensätze oder hieraus abgeleitete zweidimensionale Ansichten aus einem Bilddatenspeicher zu laden und - sofern erforderlich - für die Anzeige auf einem Bildschirm aufzubereiten.

Die View-Schicht, die ViewModel-Schicht und die Model-Schicht entsprechen hierbei dem Grundprinzip nach dem sogenannten MVVM(Model View ViewModel)-Schema, wie es an sich bei der Programmierung von Applikationen mit graphischer Nutzeroberfläche gängig ist. Ein wesentlicher Unterschied der anmeldungsgemäßen Komponenten-Architektur gegenüber dem gängigen MVVM-Schema besteht hierbei allerdings darin, dass die Komponente der ViewModel-Schicht (das "ReadingViewModel") generisch in dem Sinne ist, dass sie unabhängig von der speziellen Bilddatenart der verarbeiteten Datensätze ist. "Unabhängig" heißt hierbei, dass ein und dieselbe Komponente "ReadingViewModel" sowohl zur Anzeige und Bearbeitung von 2D-Bilddaten als auch zur Anzeige und Bearbeitung von 3D-Bilddaten eingesetzt werden kann.

Die generische Gestaltung der Komponente "ReadingViewModel" äußert dabei insbesondere darin, dass sie für sowohl für 2D-Bilddaten (und hier sowohl für Einzelbilder als auch für Keyimages, Photos, 2D-Bildsequnezen, Videos, etc.) als auch für 3D-Bilddaten jeweils einen gleichen Satz von Funktionen für die Steuerelemente der dortigen GUI-Seite definiert. Insbesondere wird durch die Komponente "ReadingViewModel" für beide gegebenenfalls vorgesehenen Komponenten "ReadingView2D" und "ReadingView3D" jeweils ein gleicher Satz von Funktionen "Play", "Stop", "Key", "+" (Zoom In), "-" (Zoom Out) etc. für Schaltflächen (Buttons) der jeweiligen GUI-Seite definiert, wobei jede dieser Funktionen im Rahmen der Komponente "ReadingViewModel" für verschiedene Bilddatenarten mit analoger (also stets quasi gleicher) Semantik belegt ist.

Beispielsweise lässt die Funktion "Play" bei 2D-Bildsequenzen die Einzelbilder nach Art einer Dia-Show-Funktion im zeitlichen Wechsel nacheinander erscheinen. Entsprechend spielt diese Funktion bei Videodaten des Videos ab. Bei 3D-Bilddatensätzen bewirkt die Funktion "Play" eine Drehung der dreidimensionalen Bildinformation.

Die Funktion "Key" löst dagegen die Erzeugung eines Keyimage aus, das vorzugsweise in JPEG verwandelt und abgespeichert wird, wobei wiederum die Art des zugrundeliegenden Datensatzes ohne Einfluss bleibt. Das Keyimage wird somit stets aus den gerade angezeigten Bilddaten erzeugt, egal ob es sich bei dem zugrundeliegenden Bilddatensatz um einen 2D-Bilddatensatz, ein Photo, ein (laufendes oder stehendes) Video, ein (stehender oder sich drehender) 3D-Bilddatensatz, etc. ist.

Auch die Komponente "<Tweet>ReadingModel" der Model-Schicht ist generisch in dem Sinne gestaltet, dass sie für alle Bilddatenarten wiederverwendet werden kann.

Die generische Gestaltung der Komponenten der ViewModel-Schicht und gegebenenfalls der Model-Schicht ermöglicht hierbei eine wesentliche Reduzierung des Herstellungsaufwands für die oder jede Viewing-Applikation. Insbesondere wird hierdurch eine fast automatische Generierung der Viewing-Applikation ermöglicht.

Zudem wird das herkömmliche MVVM-Modell nach der erfindungsgemäßen Lehre durch zumindest eine weitere Schicht, nämlich die Treiberschicht erweitert. Durch die zuunterst der Schichtenarchitektur ergänzte Treiberschicht mit ihren unterschiedlichen Treiberkomponenten für 2D-Bilddaten und 3D-Bilddaten wird ein flexibler - an beide Datenarten besonders gut angepasster und somit besonders effizienter - Betrieb der Viewing-Applikation ermöglicht.

Vorzugsweise umfasst die Komponenten-Architektur der Viewing-Applikation zusätzlich eine weitere Schicht, die als "Rahmenschicht" bezeichnet und der View-Schicht überlagert ist. Die Rahmenschicht umfasst eine (vorzugsweise lediglich eine einzige) Komponente. Die Komponente der Rahmenschicht (nachfolgend als "ReadingHostAdapter" bezeichnet) stellt einen generischen Ablauf-Rahmen für die durch die Komponenten der View-Schicht definierten GUI-Seiten dar. Nach außen hin präsentiert sich die Rahmenkomponente entweder als native Applikation, die selbstständig auf dem Betriebssystem des Nutzergeräts lauffähig ist, z.B. als IOS-App, Phone-Gap-Exe oder Windows8-App. Sie kann alternativ aber auch zum Ablauf in einem Web-Browser eingerichtet, und in dieser Variante beispielsweise als HTML5-Programm gestaltet sein. Die Rahmenkomponente dient somit als softwaretechnischer Adapter zwischen der oder jeder GUI-Seite und der nativen Laufzeitumgebung der Applikation, insbesondere dem Betriebssystem des Nutzergeräts oder einem Web-Browser.

Die Rahmenkomponente kann hierbei aus Nutzersicht entweder unsichtbar sein oder selbst einen übergeordneten Teil der graphischen Nutzeroberfläche der Viewing-Applikation bilden, und dabei insbesondere die angezeigte GUI-Seite - im Wortsinne - umrahmen.

Durch die zuoberst der Schichtenarchitektur ergänzte Rahmenschicht wird hierbei ermöglicht, einen Großteil der Komponenten der Viewing-Applikation bei Anpassung der Applikation an unterschiedliche Nutzergerätearten (PC, Smartphone, Tablet, etc.) unverändert wiederzuverwenden, wodurch der Herstellungsaufwand für die diversifizierten Applikationsvarianten wesentlich reduziert wird.

Im Betrieb der Viewing-Applikation richten die Komponenten der übergeordneten Schichten, insbesondere die Komponente der ViewModell-Schicht regelmäßig Anfragen an die jeweils zugeordnete Treiberkomponente, also den "ReadingService2D" oder den "ReadingServiceAgent3D". Jede dieser Anfragen ist auf die Übermittlung einer aus einem Bilddatensatz abgeleiteten und zur Anzeige aufbereiteten Bildinformation (Frame) gerichtet und wird regelmäßig von einem Bedienereignis (z.B. Maus-Event oder Touch-Event) ausgelöst. In einer vorteilhaften Ausführung ist die oder jede Treiberkomponente dabei derart eingerichtet, dass sie diese Anfragen asynchron und parallel bearbeitet. Die Treiberkomponente stellt diese komplexe Verarbeitungslogik allerdings nicht nach außen dar. Vielmehr gibt sie bei Erhalt einer Anfrage zweckmäßigerweise keine unmittelbare Rückmeldung an die anfragende Komponente zurück, sondern antwortet erst durch Rückgabe des aufgrund der Anfrage erstellten Frames. Auf diese Weise ist eine Pipeline zum Streamen der Bildinformation gebildet, bei der der Datenfluss an keiner Stelle durch aktives Warten auf die Antwort einer anderen Komponente unterbrochen wird. Somit werden ein besonders effizienter Datenfluss, mithin eine besonders gute Echtzeit-Performance erzielt.

Die zum Laden von 2D-Bilddaten eingerichtete Treiberkomponente "ReadingService2D" ist vorzugsweise dazu eingerichtet, Bilddatensätze unmittelbar ohne Vermittlung durch einen Server aus einem vorbestimmten Datenspeicher zu laden. Bei diesem Datenspeicher kann es sich - insbesondere in einem Offline-Betriebsmodus der Viewing-Applikation - um den lokalen Speicher des Nutzergeräts handeln. Im Normalbetrieb (Online-Betrieb) der Viewing-Applikation greift der "ReadingSer-vice2D" allerdings auf den Cloud-Storage einer Public Cloud zu. Hierbei nimmt der "ReadingService2D" insbesondere weder Cloud-Compute-Dienste noch andere Serverleistung in Anspruch, sondern addressiert den Cloud-Storage unmittelbar (insbesondere durch einen HTML-Request), wodurch sowohl ein besonders effizienter Datenstrom erzielt als auch in erheblichem Umfang Betriebskosten gespart werden.

Die zum Laden von 3D-Bilddaten vorgesehene Treiberkomponente "ReadingServiceAgent3D" ist dagegen vorzugsweise dazu eingerichtet, im Normalbetrieb (Online-Modus) die anzuzeigenden Frames - bei denen es sich hier wie vorstehend erwähnt um zweidimensionale Ansichten der dreidimensionalen Bildinformation, insbesondere gerenderte Bildszenen handelt - durch (applikationsexterne) Cloud-Compute-Dienste generieren zu lassen. In diesem Fall werden die 3D-Bilddaten nicht selbst von der Viewing-Applikation geladen. Vielmehr werden die 3D-Bilddaten durch die von den "ReadingServiceAgent3D" beauftragten Cloud-Compute-Diensten geladen. Der "ReadingService-Agent3D" erhält von den Cloud-Compute-Diensten nur die fertigen Frames.

Der "ReadingServiceAgent3D" ist vorzugsweise allerdings reversibel von dem Online-Modus in einen Offline-Modus schaltbar, in dem der "ReadingServiceAgent3D" den anzuzeigenden 3D-Bilddatensatz in den lokalen Speicher des Device lädt und die Frames zur Darstellung auf dem Bildschirm des Device selbst oder durch Aufruf einer applikationsinternen Servicekomponente auf dem Nutzergerät und ohne Inanspruchnahme von Cloud-Compute-Diensten aus der dreidimensionalen Bildinformation ableitet.

Die Bilddatensätze werden im Rahmen des Systems in einem Cloud-Storage einer Public-Cloud derart vorgehalten, dass auf jeden Bilddatensatz bei Vorliegen einer entsprechenden Cloud-Subscription über eine zugehörige URL unmittelbar, d.h. ohne Inanspruchnahme von Cloud-Compute-Diensten oder sonstiger Serverleistung zugegriffen werden kann. Zur Anzeige eines der im Cloud-Storage gespeicherten Bilddatensätze ist die Viewing-Applikation hierbei unter Angabe der URL dieses Bilddatensatzes als Argument aufrufbar, so dass die Viewing-Applikation den Bilddatensatz (oder im Falle von 3D-Bilddaten ggf. den ersten daraus abgeleiteten Frame) nach dem Start ohne weitere Nutzerinteraktion lädt und anzeigt.

Die Viewing-Applikation selbst wird ebenfalls in einem Cloud-Storage einer Public Cloud vorgehalten, so dass die Viewing-Applikation bei Vorliegen einer entsprechenden Cloud-Subscription über eine zugehörige URL heruntergeladen werden kann. In diesem Fall ist die Viewing-Applikation derart gestaltet, dass in der Download-Anfrage zusätzlich zu der URL der Viewing-Applikation die URL des anzuzeigenden Bilddatensatzes als Argument spezifizierbar ist. Dies hat ebenfalls die Folge, dass die Viewing-Applikation den Bilddatensatz (oder im Falle von 3D-Bilddaten ggf. den ersten daraus abgeleiteten Frame) nach dem Herunterladen und dem Start ohne weitere Nutzerinteraktion lädt und anzeigt.

Die Viewing-Applikation kann einerseits - unabhängig von den bilderzeugenden medizinischen Modalitäten - zum Befunden (Reading) der Bilddaten eingesetzt werden. Sie kann andererseits aber auch in unmittelbarem Zusammenhang mit einer Modalität zur Durchführung und Begleitung einer bildgebenden Untersuchung (Examination) eingesetzt werden.

Nachfolgend werden Ausführungsbeispiele der Erfindung anhand einer Zeichnung näher erläutert. Darin zeigen:
- FIG 1: in einem schematischen Blockschaltbild ein System zur Verwaltung und Bearbeitung von Daten einer medizinischen Einrichtung, mit mehreren Modalitäten und Nutzergeräten (auch als Endgeräte oder Devices bezeichnet) sowie mit einer mit den Modalitäten und Devices über das Internet verbundenen Public Cloud,

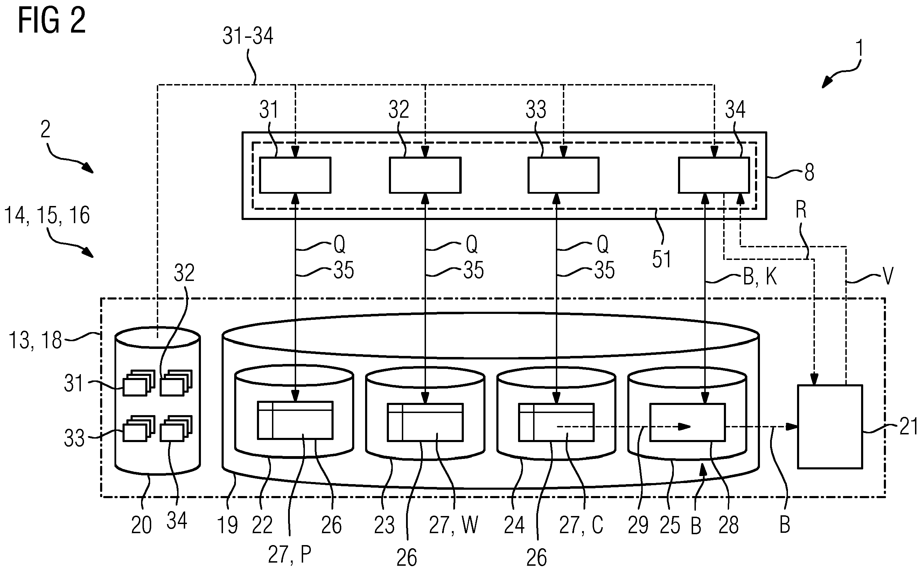

- FIG 2: in einem schematischen Blockschaltbild den softwaretechnischen Aufbau des Systems, wonach das System für verschiedene Arten von Daten der medizinischen Einrichtung in einem Cloud Storage der Public Cloud jeweils spezifisch zugeordnete Speicherbereiche (Hubs) aufweist, nämlich einen Patient-Hub für Patientendatensätze, einen Worklist-Hub für Aufgaben, einen Context-Hub für Kontextdatensätze und einen Image-Hub für Bilddatensätze, und wobei das System auf jedem Device für jeden Hub eine zugeordnete Applikation zum Zugriff auf die in diesem Hub gespeicherten Datensätze enthält, wobei in den drei erstgenannten Hubs jeweils ein Tabellenspeicher (Table Storage) angelegt ist, in dem zu jedem Datensatz des Hubs ein zugeordneter Listeneintrag (Tweet) enthalten ist, und wobei jede Applikation dazu eingerichtet, den Tabellenspeicher des zugehörigen Hubs zu durchsuchen (Browsing) und eine Datensatzliste (Feed) ausgewählter Tweets zu extrahieren und anzuzeigen, und wobei die dem Image-Hub zugeordnete Applikation zur Anzeige (Viewing) der Bilddatensätze eingerichtet ist,
- FIG 3: in schematischer Darstellung eine GUI-Seite einer zum Ablauf auf einem Smartphone vorgesehene Benutzeroberfläche (GUI) der dem Image-Hub zugeordneten Viewing-Applikation, die zur Darstellung der Bildinformation eines Bilddatensatzes dient,
- FIG 4: in Darstellung gemäß FIG 3 eine zum Ablauf auf einem Personal-Computer, Notebook oder Tablet-Compu-ter vorgesehene Variante der dortigen ViewingApplikation, und
- FIG 5: in einem schematischen Blockdiagramm die Komponenten-Architektur der dem Image-Hub zugeordneten Vie-wing-Applikation.

Einander entsprechende Teile und Größen sind in allen Figuren stets mit gleichen Bezugszeichen versehen.

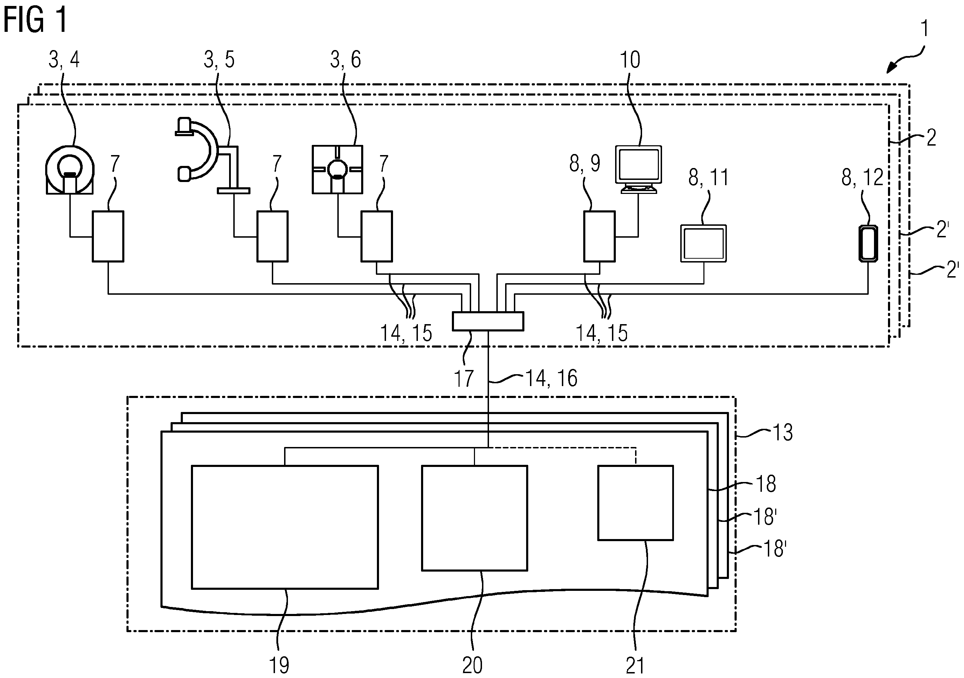

FIG 1 zeigt in grober schematischer Vereinfachung ein System 1 zur Verwaltung und Bearbeitung von medizinischen Daten einer medizinischen Einrichtung 2, bei der es sich beispielsweise um eine Klinik handelt.

Hardwareseitig umfasst das System 1 eine Anzahl von Modalitäten 3, also bildgebenden, medizinischen Untersuchungsgeräten der Einrichtung 2. So umfasst das System 1 in der beispielhaften Darstellung gemäß FIG 1 einen Computertomographen 4, ein C-Bogengerät 5 und einen Magnetresonanztomographen 6. Jeder Modalität 3 ist ein (Steuer- und Auswerte-)Rechner 7 zugeordnet.

Weiterhin umfasst das System 1 hardwareseitig eine Anzahl von Nutzer- oder Endgeräten (nachfolgend Devices 8) der Einrichtung 2, die zur Anzeige und Bearbeitung von Daten dienen. In dem vereinfachten Beispiel gemäß FIG 1 umfassen die Devices 8 einen Personalcomputer 9 mit angeschlossenem Bildschirm 10, ein Tablet 11 sowie ein Smartphone 12.

Als außerhalb der Einrichtung 2 angeordneten Bestandteil umfasst das System 1 eine Public Cloud 13. Als Public Cloud 13 wird im Rahmen des System 1 beispielsweise der von der Firma Microsoft unter der Bezeichnung "Windows Azure" angebotene Dienst genutzt. Abweichend hiervon können im Rahmen der Erfindung allerdings auch eine andere Public Cloud oder eine Kombination mehrerer Public Clouds (gegebenenfalls auch von unterschiedlichen Anbietern) als Public Cloud 13 herangezogen werden.

Die Public Cloud 13 ist über ein (Datenübertragungs-)Netz 14 mit den einrichtungsinternen Komponenten des Systems 1, also den Modalitäten 3 und Devices 8 verbunden. Diese Netzverbindung ist innerhalb der Einrichtung 2 durch ein Intranet 15 der Einrichtung 2 gebildet, das beispielweise als sogenanntes Local Area Network (LAN) auf Basis kabelgebundener Ethernet-Technologie und/oder als kabelloses Wireless Local Area Network (WLAN) aufgebaut ist. Außerhalb der Einrichtung 2 ist das Netz 14 durch das Internet 16 gebildet. An der Schnittstelle zwischen dem Intranet 15 und Internet 16 ist in üblicher Weise eine Firewall 17 angeordnet.

Die von der Public Cloud 13 im Rahmen des Systems 1 zur Verfügung gestellten Dienste sind durch einen als Subscription 18 bezeichneten Nutzungsvertrag festgelegt. Die Subscription 18 regelt dabei, welche Hardware- und Softwarebestandteile der Public Cloud 13 den einrichtungsinternen Komponenten des Systems 1 zugänglich sind. Mit dem Begriff Subscription 18 wird daher nachfolgend derjenige Teil der Public Cloud 13 bezeichnet, der der Einrichtung 2 im Rahmen des Systems 1 exklusiv zugeordnet ist. Andere Bereiche der Public Cloud 13 können - wie in FIG 1 angedeutet - im Rahmen weiterer Subscriptions 18' weiteren medizinischen Einrichtungen 2' zugewiesen sein. Jede Einrichtung 2,2' hat hierbei ausschließlich Zugang zu den ihr gemäß ihrer Subscription 18,18' zugewiesenen Daten und Diensten, nicht aber zu den Daten und Diensten anderer Einrichtungen 2 bzw. 2'. Die Public Cloud 13 ist in diesem Sinne "mandantenfähig" (multi-tenant).

Gemäß der Subscription 18 sind dem System 1 der Einrichtung 2 innerhalb der Public Cloud 13
- ein nachfolgend als Cloud Storage 19 bezeichneter Datenspeicher,
- ein nachfolgend als App Store 20 bezeichneter Speicher für Applikationen (Anwendungssoftware) sowie • Cloud-Compute-Dienste 21
zur Verfügung gestellt. Der Cloud Storage 19 dient zur persistenten Speicherung der Daten der Einrichtung 2. Der App Store 20 stellt Applikationen, Treiber und sonstige Peripheriesoftware wie beispielsweise Konfigurationsdateien und Vorlagen (Templates) zur Verfügung, die von den einrichtungsinternen Komponenten des Systems 1, also den Modalitäten 3 und den Devices 8 heruntergeladen werden können und im Betrieb des Systems 1 auf den Modalitäten 3 (genauer gesagt den zugeordneten Rechnern 7) bzw. auf den Devices 8 ablaufen. Die Cloud-Compute-Dienste 21 werden im Rahmen des Systems lediglich optional für alle Rechenoperationen herangezogen, die nicht auf den Modalitäten 3 oder den Devices 8 selbst vorgenommen werden. Letzteres betrifft insbesondere die rechenleistungsintensive Aufbereitung von 3D-Bilddaten (Volumendaten) der jeweiligen Modalität 3 für die Speicherung (Preprocessing) und/oder die Ableitung von gerenderten Ansichten V (Szenen oder Scene-Graphs) für die zweidimensionale Visualisierung (Bildsynthese, Volume Rendering) solcher Volumendaten.

Die im Rahmen der Einrichtung 2 zu verwaltenden und zu bearbeitenden Daten umfassen gemäß FIG 2 Patientendatensätze P, Aufgaben W, Kontextdatensätze C und Bilddatensätze B, wobei diese Daten im Einzelnen jeweils die vorstehend näher erläuterten Inhalte haben. Bilddatensätze B können hierbei medizinische 2D-Bilddaten (inklusive 2D-Bildsequenzen, Photos und Videos, Dokumente) oder 3D-Bilddaten (Volumendaten) oder eine sonstige Bild-, Ton-, Graphik- oder Textinformation enthalten.

Gemäß FIG 2 ist für jede dieser verschiedenen Datenarten (Ressourcen) innerhalb des Cloud Storage 19 jeweils ein separater Speicherbereich angelegt, der nachfolgend als (Ressource-)Hub bezeichnet ist. Im Einzelnen sind in dem Cloud Storage 19 somit
- ein (Patient-)Hub 22 zur Speicherung der Patientendatensätze P,
- ein (Worklist-)Hub 23 zur Speicherung der Aufgaben W,
- ein (Context-)Hub 24 zur Speicherung der Kontextdatensätze C sowie
- ein (Image-)Hub 25 zur Speicherung der Bilddatensätze B angelegt.

Jeder der Hubs 22-24 enthält jeweils einen Tabellenspeicher (nachfolgend als Table Storage 26 bezeichnet).

Im Rahmen des Systems 1 wird für jeden gespeicherten Datensatz in dem Table Storage 26 des jeweils zugeordneten Hubs 22-24 ein Listeneintrag angelegt, der nachfolgend als Tweet 27 bezeichnet ist.

Bei Datensätzen, die ihrer Natur nach aus vergleichsweise wenigen, diskreten Angaben bestehen, ist dabei der gesamte Datensatz oder zumindest ein Teil desselben als Tabellen- oder Listeneintrag (nachfolgend Tweet 27) im Table Storage 26 des jeweiligen Hubs 22-24 gespeichert. Dies betrifft im Beispiel gemäß FIG 2 die Patientendatensätze P, die Aufgaben W, sowie die Kontextdatensätze C. Anstelle des Table Storage 26 kann der Worklist-Hub 23 zur Speicherung der Aufgaben W nach einem First-in-first-out-Prinzip allerdings auch einen sogenannten Queue-Storage aufweisen.

Zur Speicherung der Bilddatensätze B, die in der Regel im Wesentlichen aus einem größeren und nicht-alphanumerischen Datenblock gebildet sind, ist im Image Hub 25 anstelle des oder zusätzlich zu dem Table-Storage 26 ein sogenannter Blob-Storage 28 vorgesehen, in dem die Bilddatensätze B als "Binary Large Objects (BLOB)" in nicht weiter strukturierter Form gespeichert sind. Zu den in dem Image Hub 25 gespeicherten Bilddatensätzen B ist in dem Kontext Hub 24 jeweils ein Kontextdatensatz C als Tweet 27 hinterlegt. Dieser Tweet 27 enthält zumindest einen URL 29 (Uniform Ressource Locator), der den Speicherort des Bilddatensatzes B im Blob-Storage 28 des Image Hubs 25 bezeichnet.

Für jeden der Hubs 22-25 wird im App Store 20 eine Applikation 31-34 zur Verfügung gestellt, die jeweils selektiv zur Darstellung und zur Bearbeitung der in dem jeweils zugeordneten Hub 22-25 gespeicherten Datensätze dient. Jede der Applikationen 31-34 repräsentiert somit den zugeordneten Hub 22-25 auf der Ebene des Device 8. Die Applikationen 31-34 sind daher - in Anlehnung an die Ressource-Hubs - auch als "Application-Hubs" bezeichnet.

Im Einzelnen umfassen die im App Store 20 zur Verfügung gestellten Applikationen somit
- eine Applikation 31 zur Verwaltung und Bearbeitung der in dem Patient-Hub 22 gespeicherten Patientendatensätze P,
- eine Applikation 32 zur Verwaltung und Bearbeitung von Aufgaben W,
- eine Applikation 33 zur Verwaltung und Bearbeitung von Kontextdatensätzen C, sowie
- eine Applikation 34 zur Anzeige und Bearbeitung der in dem Image-Hub 25 gespeicherten Bilddatensätze B.

Die Applikationen 31-34 können aus dem App Store 20 auf jedes der Devices 8 heruntergeladen und dort zur Ausführung gebracht werden. Sofern das System 1 - wie in FIG 1 dargestellt - unterschiedliche Arten von Devices (z.B. Personal-Computer 9, Tablet-Computer 11 und/oder Smartphones 12) umfasst, werden in dem App Store 20 jeweils verschiedene Versionen der Applikationen 31-34 zur Verfügung gestellt, die zum Ablauf auf dem jeweiligen Device 8 angepasst sind. Vorzugsweise werden die Applikationen 31-34 und deren Varianten allerdings nicht in vorgefertigtem Zustand im App-Store 20 vorgehalten. Vielmehr ist im App-Store 20 zumindest für die funktionsähnlichen (Browser-)Applikationen 31-33 und die (Viewing-)Applikation 34 jeweils ein Applikations-Template in Form einer XML-Datei hinterlegt, aus dem die jeweilige Applikation 31-34 bei Anforderung durch eines der Devices 8 mittels eines Applikations-Konfiguarators anhand von übermittelter Konfigurationsinformation automatisch generiert wird. Alle Applikationen 31-34 sind als Rich-Client-Applikationen konzipiert, die ohne vorgeschalteten Server, uns insbesondere auch ohne Interaktion mit der Public Cloud 13 lauffähig und funktionsfähig sind.

Beispielsweise werden die Applikationen 31-34 zum Ablauf auf dem Tablet-Computer 11 und dem Smartphone 12 in Form einer an den jeweiligen Gerätetyp angepassten App zur Verfügung gestellt, während die für den Personal-Computer 9 zur Verfügung gestellte Versionen der Applikationen 31-34 für den Ablauf in einem Web-Browser konzipiert sind.

Im Vergleich zu einer herkömmlichen IT-Struktur für die Verwaltung von medizinischen Daten übernehmen der Patient Hub 22 und die zugeordnete Applikation 31 aus Sicht des Nutzers etwa die Funktion eines herkömmlichen Krankenhaus-Informationssystems (HIS). Insbesondere sind die gesamten personenbezogenen (und datenschutzrelevanten) Patientendaten in dem Patient-Hub 22 aufgenommen, der somit für den einzelnen Patienten eine elektronische Patientenakte abbildet. Die übrigen Hubs 23-25 enthalten vorzugsweise keine Information, die aus sich heraus einem bestimmten Patienten zuordenbar wäre (Privacy Information). Der Worklist-Hub 23 und die zugeordnete Applikation 32 stellen sich dem Nutzer im Wesentlichen als Entsprechung zu einem herkömmlichen Radiologie-Informationssystems (RIS) dar, indem sie die innerhalb der Einrichtung 1 zur Durchführung anstehenden Aufgaben auflisten. Der Context-Hub 24 und die zugeordnete Applikation 33 übernehmen aus Nutzersicht etwa die Funktion eines herkömmlichen PACS, indem dort die zur Auffindung und Verarbeitung von Bilddatensätzen B erforderliche Information hinterlegt und recherchierbar ist. Der Image-Hub 25 und die zugeordnete Applikation 34 übernehmen schließlich etwa die Funktion eines herkömmlichen AV-Systems.

Obwohl die in den Hubs 22-25 und den zugeordneten Applikationen 31-34 enthalten Funktionen hochgradig miteinander verknüpft sind, ist jeder der Hubs 22-25 zusammen mit der jeweils zugeordneten Applikation 31-34 auch unabhängig von den jeweils anderen Hubs 22-25 und deren Applikationen 31-34 betreibbar. So kann beispielsweise auf einen in dem Image-Hub 25 enthaltenen Bilddatensatz B auch ohne den zugehörigen Kontextdatensatz C des Context-Hubs 24 zugegriffen werden, wenn der dem Image-Hub 25 zugeordneten Applikation 34 der URI 29 des anzuzeigenden Bilddatensatzes B auf andere Weise verfügbar gemacht wird, z.B. per E-Mail, SMS oder Twitter. Ferner können die Patientendaten P auch außerhalb der Public Cloud 13 vorgehalten werden, da der Patient-Hub 22 für die Funktion der übrigen Hubs 23-25 und der zugeordneten Applikationen 32-34 nicht erforderlich ist.

Bei der dem Patient-Hub 22 zugeordneten Applikation 31 handelt es sich um eine Browser-Applikation, die den Table Storage 26 des Patient-Hubs 22 nach Tweets 27 durchsucht, die einem bestimmten, von einem Nutzer vorgebbaren Suchbegriff entsprechen. Von dem Patient-Hub 22 wird hierbei auf eine Suchanfrage Q (FIG 2) der Applikation 31 hin eine nachfolgend als Feed 35 bezeichnete Datensatzliste, die die der Suchanfrage Q entsprechenden Tweets 27 enthält, an die Applikation 31 zurückgeliefert. Die Applikation 31 zeigt den erhaltenen Feed 35 auf dem Device 8 GUI-Seite einer (nicht näher dargestellten) graphischen Benutzeroberfläche (GUI) an. Ein Tweet 27 kann aus diesem angezeigten Feed 35, z.B. durch Antippen mit dem Finger aktiviert werden. In diesem Fall wird der Inhalt des Tweets 27 in einer weiteren GUI-Seite im Detail dargestellt. Die Applikationen 32 und 33 haben jeweils eine der Applikation 31 entsprechende Browsing-Funktion.

Jede der Applikationen 31-33 ist dazu eingerichtet, Feeds 35 auf Anweisung des Nutzers aus dem zugehörigen Hub 22-24 in einen lokalen Speicher des Device 8 zu externalisieren, so dass dieser Feed 35 und die darin enthaltenen Tweets 27 durch die Applikation 31-33 auch ohne Netzanbindung mit der Public Cloud 13 angezeigt und/oder bearbeitet werden können. Weiterhin ist jede Applikation 31-33 dazu eingerichtet, auf entsprechenden Befehl des Nutzers externalisierte Feeds 35 oder Tweets 27 an eine andere Instanz derselben oder einer anderen Applikation 31-34 zu übermitteln. Für die geräteinterne Übermittlung der Feeds 35 oder Tweets 27 ist im Speicher des Device 8 ein als "Exchangeboard" bezeichneter lokaler Speicherbereich eingerichtet, auf den alle Instanzen der Applikationen 31-34 gemeinsam zugreifen. Zum Austausch über das Exchangeboard werden die auszutauschenden Feeds 35 oder Tweets 27 in XML/JSON-Files serialisiert, die im lokalen Speicher des Devices 8 abgelegt und anschließend wieder deserialisiert werden. Zum Austausch von Feeds 35 und Tweets 27 zwischen verschiedenen Devices 8 weisen die Applikationen 31-34 bevorzugt Schnittstellen zu Twitter oder einem E-Mail-Programm auf.

Die Applikation 34 dient dagegen zur Anzeige ("Viewing") des nicht-alphanumerischen Inhalts eines im Image-Hub 25 gespeicherten Bilddatensatzes B. Die Applikation 34 wird im Normalfall aus einer der Applikationen 31-33 heraus gestartet. Insbesondere wird die Applikation 34 aus der dem Context-Hub 24 zugeordneten Applikation 34 gestartet, indem dort ein angezeigter Tweet 27 durch den Finger, eine Maus oder dgl. - beispielsweise nach Art eines "Doppelklicks" - aktiviert wird. In diesem Fall wird die Applikation 34 gestartet, wobei der Applikation 34 der aus dem Tweet 27 extrahierte URL des zugehörigen Bilddatensatzes B als Argument mitgegeben wird. Dies hat zur Folge, dass die Applikation 34 den Bilddatensatz nach dem Start ohne weitere Nutzerinteraktion aus dem Image-Hub 25 lädt und anzeigt. Bilddaten B sind im Image-Hub 25 insbesondere im JPEG-Format hinterlegt, Volumendaten vorzugsweise im DICOM-Format. Die gegebenenfalls von der Public-Cloud 13 an die Applikation 34 gelieferten Ansichten V (Scene-Graphs) von Volumendaten werden vorzugsweise im JPEG-Format erstellt. Zusätzlich oder alternativ können im Image-Hub 25 aber auch Bilddaten und Dokumente in anderen Datenformaten, z.B. webm, mp4, pdf oder dz abgespeichert sein.

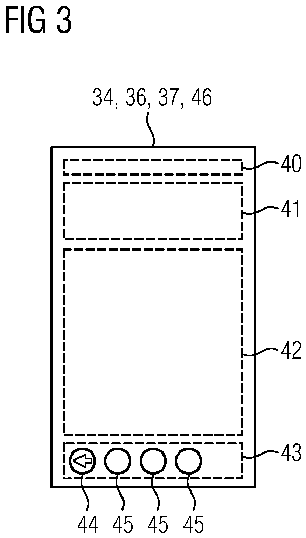

Hierfür umfasst die Applikation 34 eine in FIG 3 dargestellte (GUI-)Seite 36 einer graphischen Benutzeroberfläche 37.

In der exemplarischen Ausführung gemäß FIG 3 weist die GUI-Seite 36 im Wesentlichen vier Anzeigebereiche unterschiedlicher Funktion auf, nämlich eine Kopfzeile 40, ein Überschriftenfeld 41, ein Anzeigefeld 42 und eine Befehlsleiste 43. In der Kopfzeile 40 ist der Image-Hub 25 namentlich benannt, dem die Applikation 34 zugeordnet ist. Die Kopfzeile 40 somit z.B. mit dem Wort "Image Hub" beschriftet. Des Weiteren ist in der Kopfzeile 40 vorzugsweise die Einrichtung 2 benannt, der das System 1 zugeordnet ist.

In dem Überschriftenfeld 41 ist beispielsweise die Art des angezeigten Bilddatensatzes B spezifiziert (z.B. als "2D-Viewing", 3D-Viewing", "Media-Viewing", "Document--Viewing", etc.). Im Anzeigefeld 42 wird der visuelle Inhalt des Bilddatensatzes B, also beispielsweise das Bild, Volumen, Video oder Textdokument angezeigt. Die Befehlszeile 43 enthält eine Schaltfläche 44, auf deren Betätigung hin die Applikation 34 die angezeigte GUI-Seite 36 schließt und zu einer GUI-Seite der zuletzt verwendeten Browser-Applikation 31-33 zurückwechselt. Weitere Schaltflächen 45 dienen zur Beeinflussung der Anzeige, beispielsweise zum Vergrößern (Zoom In) oder Verkleinern (Zoom Out) des im Anzeigefeld 42 angezeigten Dateninhalts, Starten (Start) und Beenden (Stop) eines Bildlaufs oder einer Bewegtbild-Animation, Erzeugung eines Keyimage (Key), Bild- und/oder Tonaufnahme (Rec) mittels Kamera bzw. Mikrofon des Device 8, etc.. Außerdem kann eine Schaltfläche 45 zum Löschen eines Bilddatensatzes B vorgesehen sein.

Anstelle der gemeinsamen Seite 36 zur Anzeige von Bilddaten aller Bilddatenarten kann die Applikation 34 auch verschiedene GUI-Seiten umfassen, die jeweils zur Anzeige von Bilddaten einer bestimmten Bilddatenart (Bild, Volumen, Video, Dokument, etc.) eingerichtet sind. Vorzugsweise umfasst die Applikation 34 zumindest zwei GUI-Seiten 36, von denen eine zur Anzeige von 2D-Bilddaten (inklusive Photos, 2D-Bildsequenzen und Videos) und die andere zur Anzeigen von 3D-Bilddaten dient.

Übergeordnet zu den GUI-Seiten 36 umfasst die Applikation 34 einen Rahmen 46. Dieser Rahmen 46 bildet bildet einen Adapter zu der nativen Laufzeitumgebung des jeweiligen Devices 8, in dem die Applikation 34 abläuft, also z.B. zu dem Betriebssystem oder einem Webbrowser. Der Rahmen 46 dient des Weiteren als Ablaufumgebung oder Container, in dem mehrere Instanzen der GUI-Seiten 36 nebenläufig ausgeführt werden können. In dem Ausführungsbeispiel gemäß FIG 3 wird stets nur eine dieser Instanzen angezeigt, während die anderen im Hintergrund laufen. Der Rahmen 46 kann optional einen Rahmen der Benutzeroberfläche 37 zur Verfügung stellen.

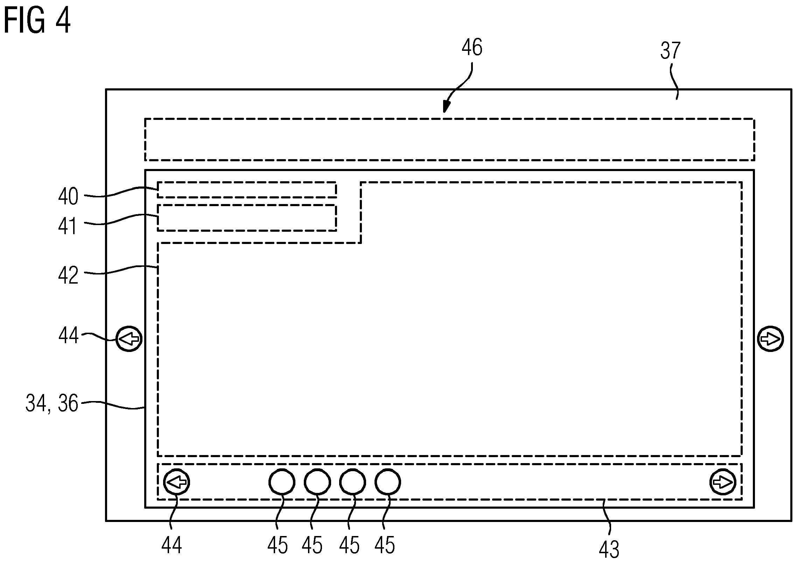

FIG 4 zeigt eine zur Anzeige auf einem großformatigen Bildschirm optimierte Version der GUI-Seite 36 der Applikation 34, bei der das Anzeigefeld 42 nahezu die gesamte Bildschirmfläche des Device 8 (hier zum Beispiel in Form des Tablet-Computers 11) ausfüllt.

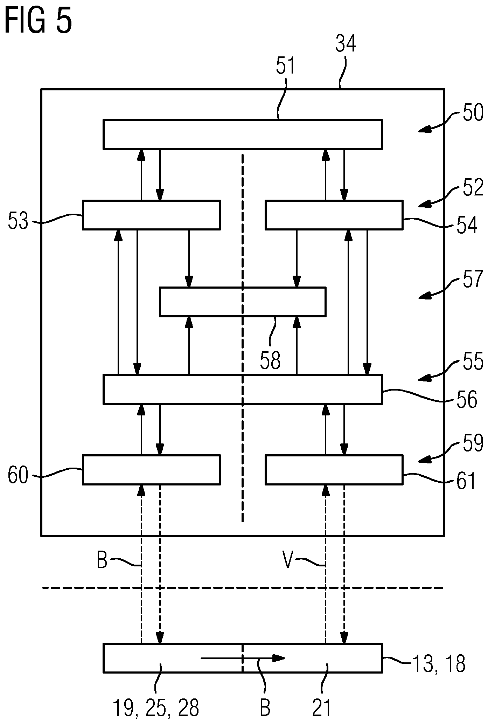

In FIG 5 ist schematisch ein Modell der Komponenten-Architektur der Applikationen 34 dargestellt. Der Darstellung ist zu entnehmen, dass die Applikation 34 aus Komponenten gebildet ist, die in fünf Schichten angeordnet sind.

Die höchste Schicht dieser Architektur ist als Rahmenschicht 50 bezeichnet. Diese Rahmenschicht 50 ist im Beispiel gemäß FIG 5 durch eine Rahmenkomponente 51 gebildet, die auch als "ReadingHostAdapter" bezeichnet ist. Die Rahmenkomponente 51 bildet - wie vorstehend näher beschrieben - den vorstehend beschriebenen Rahmen 46, also einen generischen Ablaufrahmen (Container) für die GUI-Seiten 36. Die Rahmenkomponente 51 regelt dabei insbesondere, wie die Kontrollübergabe zwischen den nebenläufig existierenden Instanzen der GUI-Seiten 36 erfolgt, wie diese Instanzen also für eine Nutzerinteraktion aktiviert und deaktiviert sowie auf dem Bildschirm ein- und ausgeblendet werden.

Die Rahmenkomponente 51 wird spezifisch in Abhängigkeit der Art des Device 8, auf dem die Applikation 34 laufen soll, ausgewählt und ist auf den Formfaktor des jeweiligen Device 8 (also die Größe und Geometrie des Bildschirms) sowie auf die in dem Device 8 vorhandenen Mittel zur Nutzerinteraktion zugeschnitten. Beispielsweise unterstützt eine zum Ablauf auf dem Tablet-Computer 11 oder Smartphone 12 vorgesehene Version der Rahmenkomponente 51 Nutzerinteraktions-Methoden, wie sie für die Nutzerinteraktion über einen Touch-Screen üblich sind (z.B. Verschieben der GUI-Seite 36 durch manuelles Wischen über den Bildschirm, Zoomen der Bildinformation durch Zwei-Finger-Operationen, Drehen der Bildinformation bei Verkippung des Devices 8 etc.). Eine für den Ablauf in einem Personal-Computer 9 ausgebildete Version der Rahmenkomponente 51 stellt dagegen bevorzugt in Anpassung an eine Nutzerinteraktion mittels Maus oder Touch-Pad grafische Steuerelemente in Form von Schaltflächen (Buttons) zur Verfügung. Die Rahmenkomponente 51 bewirkt hierbei insbesondere eine Anpassung der GUI-Seiten 36 an das jeweilige Device 8 (insbesondere an die Bildschirmgröße), und bestimmt in diesem Zusammenhang beispielsweise, ob alle Schaltflächen 45 oder nur eine bestimmte Auswahl der Schaltflächen 45 angezeigt werden.

Der Rahmenschicht 50 unterlagert ist eine View-Schicht 52. Diese View-Schicht 52 umfasst im Falle der Applikation 34 zwei Komponenten 53 und 54, die auch als "ReadingView2D" und "ReadingView3D" bezeichnet sind. Die Komponenten 53 und 54 definieren hierbei den "View", das heißt die grafische Gestaltung der GUI-Seiten 36. Die Komponente 53 ("Reading-View2D") definiert hierbei die Steuerelemente einer ersten Variante der GUI-Seite 36, die zur Anzeige von 2D-Bilddaten (inklusive Photos, Keyimages, 2D-Bildsequenzen und Videos) eingerichtet ist, hinsichtlich Art, Aussehen und Anordnung. Die Komponente 54 ("ReadingView3D") definiert dagegen in entsprechender Weise die Steuerelemente einer zweiten Variante der GUI-Seite 36, die zur Anzeige von 3D-Bilddaten eingerichtet ist.

Des Weiteren umfasst die Applikation 34 in einer ViewModel-Schicht 55 eine weitere Komponenten 56, die als "ReadingViewModel" bezeichnet ist. Diese Komponente 56 definiert für die Komponenten 53 bzw. 54 der View-Schicht 52 funktionale Eigenschaften und Befehle für die in diesen Komponenten 53 bzw. 54 definierten Steuerelemente der graphischen Benutzeroberfläche 37, insbesondere Funktionen zum Drehen, Verschieben, Zoomen der Bildinformation, etc. Die Komponente 56 der ViewModel-Schicht 95 wird hierbei auf beide Komponenten 53 und 54 der View-Schicht 52 angewendet und ist insofern generisch, also unabhängig von der Art der anzuzeigenden Bilddatensätze B gestaltet, als sie für alle Arten von anzuzeigenden Bilddatensätzen, nämlich sowohl 2D-Bilddaten als auch 3D-Bilddaten, einen gemeinsamen Satz von Funktionen (Play, Stop, Key, Zoom In, Zoom out, Rec, etc.) mit stets entsprechender Semantik zur Verfügung stellt. Die Komponente 56 belegt hierbei jede dieser Funktionen - wie vorstehend erläutert - für die verschiedenen Bilddatenarten stets mit einer vergleichbaren Semantik.

In einer Model-Schicht 57 umfasst die Applikation 34 eine Komponente 58, die beispielhaft als <Tweet>ReadingModel" bezeichnet ist. Diese Komponente 58 definiert für jede Art anzuzeigender Bilddaten, insbesondere 2D-Bilddaten zur Anzeige auf dem Bildschirm (inklusive der aus 3D-Bilddaten abgeleiteten Ansichten V) und Videos die zur Datenhaltung erforderlichen Variablen. Die Komponente 58 der Model-Schicht 57 ist ebenfalls insofern generisch gestaltet, als sie auf alle Arten anzuzeigender Bilddaten angewendet werden kann.

In einer Treiberschicht 59 umfasst die Applikation 34 zwei Treiberkomponenten 60 und 61, die auch als "ReadingService2D" bzw. "ReadingServiceAgent3D" bezeichnet sind. Die Treiberkomponenten 60 und 61 vermitteln für die übergeordneten Schichten den Zugriff auf die im Blob-Storage 28 des Image-Hub 25 hinterlegten Bilddatensätze B. Des Weiteren bereiten die Treiberkomponenten 60 und 61 die anzuzeigenden Bilddatensätze B auf, indem sie aus diesen Bilddatensätzen B Bilddaten (Frames) zur unmittelbaren Darstellung auf dem Bildschirm des Devices 8 ableiten und über die Komponente 56 der ViewModel-Schicht 55 der jeweiligen GUI-Seite 36 zuleiten.

Die Treiberkomponente 60 ("ReadingService2D") dient hierbei zum Laden von 2D-Bilddaten. Wie aus den FIG 2 und 5 hervorgeht, greift die Treiberkomponente 60 in einem Online-Modus zum Laden der Bilddatensätze B - ohne Inanspruchnahme der Cloud-Compute-Dienste 21 oder sonstiger Serverleistung - mit einem HTML-Request unmittelbar auf den Blob-Storage 28 des Image-Hubs 25 zu. Die Treiberkomponente 60 kann allerdings des Weiteren auch in einem Offline-Modus betrieben werden, in dem sie den anzuzeigenden Bilddatensatz B aus dem lokalen Speicher des Devices 8 einliest. Die Treiberkomponente 60 nimmt des Weiteren eine Aufbereitung der 2D-Bilddaten zur Anzeige auf dem Bildschirm des Device 8 vor, indem sie einen anzuzeigenden Bildausschnitt auswählt oder berechnet, die Bildinformation auf entsprechende Nutzeranforderung dreht, auf Nutzeranforderung bestimmte Bildbearbeitungsalgorithmen (z.B. Kantenschärfung oder Fensterung) auf die Bilddaten anwendet, etc.

Die Treiberkomponente 61 ("ReadingServiceAgent3D") dient dagegen zum Laden der Bilddaten für 3D-Viewing. Im Normalbetrieb werden die anzuzeigenden 3D-Bilddaten von der Treiberkomponente 61 daher nicht unmittelbar auf das Device 8 geladen. Auch führt die Treiberkomponente 61 in dem in FIG 5 dargestellten Normalbetrieb ("Online-Modus") das numerisch aufwändige Volume Rendering nicht selbst durch. Vielmehr greift die Treiberkomponente 61 zur Ableitung der zweidimensionalen Ansichten (V in Form von "gerenderten" Bildszenen) aus der dreidimensionalen Bildinformation der 3D-Bilddatensätze B auf die Cloud-Compute-Dienste 21 zu, die die Bilddatensätze B innerhalb der Cloud 13 autonom aus dem Blob-Storage 28 des Image-Hub 25 laden und nur die fertigen "gerenderten" Ansichten V an die Treiberkomponente 61 zurückgegeben.

Allerdings kann die Treiberkomponente 61 in optionaler Ausgestaltung auch in einem "Offline-Modus" betrieben werden, in dem sie die anzuzeigenden 3D-Bilddatensätze B - insbesondere aus einem lokalen Speicher des Device 8 - lädt und die zweidimensionalen Ansichten V lokal erzeugt.

Beide Treiberkomponenten 60 und 61 verarbeiten Anfragen durch die Komponente 56 der ViewModel-Schicht 55 asynchron und parallel. Im Sinne eines möglichst effizienten Datenflusses sind die Treiberkomponenten 60 und 61 derart gestaltet, dass sie auf Anfragen nicht unmittelbar antworten, sondern sich erst mit dem Ergebnis, also dem anfragegemäß erstellten Frame zurückmelden. Ebenfalls im Sinne einer möglichst hohen Effizienz der Datenbearbeitung und -übertragung ist die Treiberkomponente 61 darüber hinaus derart gestaltet, dass sie für jede zu erstellende Ansicht V nach einem Single-Instruction-Per-Frame-Prinzip nur eine einzige Nachricht (per HTTP-Request R) an die Cloud-Compute-Dienste 21 absetzt. Die Treiberkomponente greift hierzu auf eine breite, singuläre Anwender-Programmier-Schnittstelle (API) zu, die nur eine einzige, alle nötigen Parameter tragenden Methode umfasst.

Die Funktionalität zur Anzeige der 3D-Bilddaten ist eine optionale Funktion der Applikation 34, die auf Nutzeranforderung (On-Demand) oder im Rahmen einer Lizenzoption reversibel zuschaltbar ist. Entsprechend kann die Applikation 34 auch ohne die Komponente 54 und die Treiberkomponente 61 betrieben werden. Im Falle einer entsprechenden Nutzeranforderung werden die Komponente 54 und die Treiberkomponente 61 hierbei nachgeladen oder aktiviert.

Die Applikation 34 kann auf dem Device 8 fest installiert sein oder im App-Store 20 zum Download angeboten werden. In ersterem Fall wird die Applikation 34 unter Angabe des URL 29 eines anzuzeigenden Bilddatensatzes B als Argument aufgerufen. In dem letzteren Fall ist die Applikation 34 derart gestaltet, dass in der Download-Anfrage zusätzlich zu dem URL der Applikation 34 der URL 29 des anzuzeigenden Bilddatensatzes B als Argument spezifizierbar ist.

In beiden Fällen wird durch die Rahmenkomponente 51 der als Argument spezifizierte URL 29 an die Komponente 56 der ViewModel-Schicht 55 gegeben. Hier wird anhand des URL 29 die Art des anzuzeigenden Bilddatensatzes B bestimmt. In Abhängigkeit der ermittelten Bilddatenart wählt die Komponente 56 die passende Komponente 53,54 der View-Schicht 52 und die passende Treiberkomponente 60,61 der Treiberschicht 59 aus.

Bei 2D-Bilddaten (mit Ausnahme von Videostreams, die von der Komponente 56 unmittelbar gesteuert werden) wählt die Komponente 56 somit die Treiberkomponente 60 aus, während sie bei 3D-Bilddaten die Treiberkomponente 61 auswählt. Des Weiteren veranlasst die Komponente 56 - insbesondere durch entsprechende Ansteuerung der Treiberkomponente 60,61 - das Laden der anzuzeigenden Bilddaten. Somit werden durch die Applikation 34 der Bilddatensatz B oder im Falle von 3D-Bilddaten ggf. die erste daraus abgeleitete Ansicht V nach dem Start der Applikation 34 ohne weitere Nutzerinteraktion geladen und anzeigt.

Um die Applikation 34 herunterzuladen, wird vorzugsweise zunächst die für das Device 8 geeignete Rahmenkomponente 51 aus dem App Store 20 heruntergeladen. Die Rahmenkomponente 51 dient hierbei auch als Applikations-Konfigurator, indem sie anhand eines als XML-Datei im App-Store 20 vorgehaltenen Applikations-Template die benötigten Komponenten der untergeordneten Schichten nachlädt.

Mit den Funktionen "Key" (zur Erzeugung eines Keyimage K) und "Rec" (zur Bild- und/oder Tonaufnahme mittels Kamera bzw. Mikrofon des Device 8) ermöglicht die Applikation 34 die Erzeugung neuer Bilddatensätze B. Diese werden durch die Komponente 56 durch einen HTML-POST-Befehl direkt - ohne zwischengeschaltete Cloud-Compute-Dienste 21 oder sonstige Serverleistung - in den Image-Hub 25 geladen.

Die Erfindung wird an den vorstehend beschriebenen Ausführungsbeispielen besonders deutlich, ist gleichwohl auf diese aber nicht beschränkt. Vielmehr können weitere Ausführungsformen der Erfindung aus der vorliegenden Beschreibung und den Ansprüchen abgeleitet werden. Insbesondere können die anhand der Ausführungsbeispiele beschriebenen Einzelmerkmale der Erfindung auch in anderer Weise kombiniert werden, ohne von der Erfindung abzuweichen.

In den nachfolgenden Anhängen sind Beispiele für eine konkrete Implementierung der - hier in einer Komponente zusammengefassten - View-Schicht 52 ("ReadingView2D3D.html") und der Komponente 56 ("ReadingViewModel.js) der ViewModel = Schicht 55 aufgeführt.

### Anhang 1: Beispielhafte Implementierung der View-Schicht 52 - "ReadingView2D3D.html"

```
<!DOCTYPE html>
 <html>
 <head>
  <title>Syngo 2D/3D Reading-Device-App</title>
  <meta name="demopage" content="1" /><meta http-equiv="X-
  UA-Compatible" content="IE=edge" />
  <script src="js/jquery-1.7.2.js"></script>
  <link rel="stylesheet" href="css/ReadingView2D3D.css" />
  <script src="js/uInt8Array_getter_setter.js"></script>
  <script src="js/StringFormatter.js"></script>
  <script src="js/Point.js"></script>
  <script src="js/InstanceInfo.js"></script>
  <script src="js/ReadingServiceAgent3D.js"></script>
  <script src="js/arrayBufferToDataUri_Common.js"></script>
  <script src="js/uInt8Array_IE9.js"></script>
  <script src="js/ReadingViewModel.js"></script>
  <script src="js/ReadingServiceAgent2D.js"></script>
  <script type="text/javascript"
  src="js/FeatureDetectCanvas.js"></script>
  <script src="js/ReadingService2D.js"></script>
  </head>
  <body>
 <div id="workspace">
     <div id="NavigationAppBar" >
           <input type="image" on-
           click="GUI_ViewDetail_Click()"
           src="images/ApplicationBar.Check.png" id="qualityButton">
           <input type="image" on-
           click="GUI_SwitchAppBar_Click()"
           src="images/ApplicationBar.Refresh.png"
           id="switchAppBarButton">
     </div>
     <div id="ViewingAppBar">
           <input type="image" on-
           click="GUI_ApplicationBarIconButton_ROTATE_Click()"
           src="images/ApplicationBar.Rotate.png" id="rotateButton">
           <input type="image" on-
           click="GUI_ApplicationBarIconButton_WINDOWING_Click()"
           src="images/ApplicationBar.Windowing.png"
           id="windowingButton">
           <input type="image" on-
           click="GUI_ApplicationBarIconButton_START_Click()"
           src="images/ApplicationBar.Start.png" id="startButton">
           <input type="image" on-
           click="GUI_ApplicationBarIconButton_2D_Click()"
           src="images/ApplicationBar.2D.png" id="zweiDButton">
           <input type="image" on-
           click="GUI ApplicationBarIconButton_SaveKeyIma_Click()"
           src="images/ApplicationBar.Save.png" id="saveKeyImaButton1">
           <input type="image" on-
           click="GUI_ApplicationBarIconButton_Import_Click()"
           src="images/ApplicationBar.Download.png" id="importButton1">
           <input type="image" on-
           click="GUI_ApplicationBarIconButton_Export_Click()"
           src="images/ApplicationBar.Upload.png" id="exportButton1">
     </div>
     <div id="DrawingAppBar" >
           <input type="image" onclick="setColor(0,0,0)"
           src="images/black1.png" id="blackButton">
           <input type="image" onclick="setColor(0xff,0,0)"
           src="images/red.png" id="redButton">
           <input type="image" onclick="setColor(0,0xff,0)"
           src="images/green.png" id="greenButton">
           <input type="image" onclick="setColor(0,0,0xff)"
           src="images/blue.png" id="blueButton">
           <input type="image" onclick="setPen(10)"
           src="images/thick3.png" id="thickButton">
           <input type="image" onclick="setPen(5)"
           src="images/middle3.png" id="mediumButton">
           <input type="image" onclick="setPen(1)"
           src="images/thin3.png" id="thinButton">
           <input type="image" onclick="clearCanvas()"
           src="images/ApplicationBar.Cancel.png" id="clearButton">
           <input type="image" on-
           click="GUI_ApplicationBarIconButton_SaveKeyIma_Click()"
           src="images/ApplicationBar.Save.png" id="saveKeyImaButton2">
           <input type="image" on-
           click="GUI_ApplicationBarIconButton_Import_Click()"
           src="images/ApplicationBar.Download.png" id="importButton2">
           <input type="image" on-
           click="GUI_ApplicationBarIconButton_Export_Click()"
           src="images/ApplicationBar.Upload.png" id="exportButton2">
     </div>
     <div id="PerfCounter" > <p id="fps" > </p> </div>
     <div id="Image1" > <img id="msi2" src=""/> </div>
     <div id="Canvas1" > <canvas id="msi1" ></canvas> </div>
     <div id="title"> </div>
     <div id="container" style="text-align: center;"> </div>
     <div id="hotspots"> </div>
 </div>
 </body>
 </html>
```

### Anhang 2: Beispielhafte Implementierung der ViewModel-Schicht 55 - "ReadingViewModel.js"

```
 var msi1;
 var msi2;
 var context = null;
 var workspace;
 var contacts = null;
 var myViewingPage;
 var SUPPORTEDCONTACTS = 3;
 var numActiveContacts;
 var firstPointerId = 0;
 var Dragging = false;
 var gestureObject;
 var pointerSink = null;
 var canvasgroup;
 var tweetUrl;
 var tweet = "";
 var canvas;
 var contextDrawing = null;
 var workspaceDrawing;
 var currentPos, currentTool, pencil, eraser, color;
 var currentColor;
 var viewing_on = true;
 var ViewingAppBar;
 var DrawingAppBar;
 var zoomin = true;
 var smartPhoneDevice = true;
 var Tooltip Forward = "start cine loop of dzc collection dzi
 images in forward direction";
 var Tooltip_Backward = "start cine loop of dzc collection dzi
 images in backward direction";
 var Tooltip_Stop = "stop a running cine loop";
 var container;
 var viewer;
 var touchController;
 var timerCine = null;
 var counterCineLoop = 0;
 var directionCine = "";
 var DziImageFormat = ".jpg";
 var tileLevelForStamp = "7";
 var hotspotsOutput;
 var fallback;
 var fpsMeter;
 var viewport;
 var avgDelay = 0;
 var lastDraw = new Date;
 var tweetUrl;
 var tweet = "";
 var collectionDZIImaRef = 0;
 var collectionDZIImaAnz = 0;
 var state = 0; // 0 = init, 1 = loadCollectionXmlFile, 2 =
 parseCollectionXml, 3 = ready
 var srcFileToLoadType = "";
 var srcFileToLoad = "";
 var hotspots = [
 ] ;
 var myguid = (G() + G() + "-" + G() + "-" + G() + "-" + G() +
 "-" + G() + G() + G()).toUpperCase(); // Sys-
 tem.Guid.NewGuid().ToString();
 function G() {
   return (((1 + Math.random()) * 0x10000) |
   0).toString(16).substring(1)
   }
   var myInstanceInfo = new InstanceInfo(myguid);
   var contents;
   var mContent; // global values for 3d volumes in config.xml
   file
   var myStorageTypeContent = "LOCAL";
   var myStorageFormatContent = "RAW";
   var myState = "Initial";
   var myLoadStrategyMode = "Chunking";
   var myStorageType = "LOCAL";
   var myStorageFormat = "RAW";
   var mydrive = "d://"; // muessen "\\" zu "//" werden sonst
   geht das nicht in die urí!!
   var mynameUC = "-USECASE VRT -VOLUME_SWIZZLING false -MODE
   DynamicLinearSampling -SHADING true";
   var myDZMHDDataRelPath =
   "IMAGES_POPIP//Data3D//RawVolumes//VRT_AngioCarotid_279.mhd";
   var myServerBaseUri = "Application.Current.Host.Source";
   var myCloud = "blob.core.windows.net";
   var myTenantStorage = "singapore";
   var myTenantCompute = "rdemo9";
   var myProcedure = "mysingapore";
   var myAppHost = "http://singapore.blob.core.windows.net/";
   var myConfigHost = "http://singapore.blob.core.windows.net/";
   var myComputeHost = "http://rdemo9.cloudapp.net/";
   var myStorageHost =
   "http://singapore.blob.core.windows.net/";
   var myStorageHostVolumeSeries2d =
   "http://singapore.blob.core.windows.net/mysingapore/IMAGES_PO
   PIP_1747/Data3D/RawVolumes/3D_MAX_1747/";
   var myStorageHostVolumeSeries2dAnz = "1747";
   var ImageSeriesmyCloud = "blob.core.windows.net";
   var ImageSeries_myTenantStorage = "singapore4";
   var ImageSeriesmyTenantCompute = "rdemo9";
   var ImageSeries_myProcedure = "mysingapore";
   var ImageSeriesmyAppHost =
   "http://singapore.blob.core.windows.net/";
   var ImageSeriesmyConfigHost =
   "http://singapore4.blob.core.windows.net/";
   var ImageSeries_myComputeHost =
   "http://rdemo9.cloudapp.net/";
   var ImageSeriesmyStorageHost =
   "http://singapore4.blob.core.windows.net/";
   var ImageSeries_myStorageHostVolumeSeries2d =
   "http://singapore.blob.core.windows.net/mysingapore/IMAGES_PO
   PIP_1747/Data3D/RawVolumes/3D_MAX_1747/";
   var ImageSeriesmyStorageHostVolumeSeries2dAnz = "1747";
   var myDZMetaDataRelPath =
   "IMAGES_POPIP//Data3D//Luts//AngioRunOff_1.0.lut";
   var myDZInstDataRelPath =
   "IMAGES_POPIP//Data3D//RawVolumes//VRT_AngioCarotid_279.raw";
   var _config_content_type = "image";
   var mode = "2D";
   var _imaging_mode = "2D";
   var _loop_mode = "Review";
   var _Menue3dPressed = false;
   window.onload = function () {
  metro = false;
  workspace = document.getElementById("workspace");
  workspace.width = window.innerWidth;
  workspace.height = window.innerHeight;
  onDOMContentLoaded(); }
  function getTheDocumentUrlParameter(paramName) {
 var searchChars = "[\\?&]"+paramName+"=([^&#]*)";
 var regularExp = new RegExp(searchChars);
 var searchResults = regularExp.exec(window.location.href);
 if(searchResults!=null) return searchResults[1];
 else return "";
 }
 function getUrlTweetParameter_old() {
 whole=document.location.href;
 var leftside = whole.split('?')[0];
 var rightside = whole.split('?')[1];
 tweet = "";
 if (rightside != "" && rightside != null) {
    tweet = getTheDocumentUrlParameter("tweet");
 }
 return (tweet);
 function getUrlTweetParameter() {
 //alert("getUrlTweetParameter") ;
 whole=document.location.href;
 var leftside = whole.split('?')[0];
 var rightside1 = whole.split('?')[1];
 var rightside = whole.substring(whole.indexOf('?'),
 whole.length);
 tweet = "";
 if (rightside != "" && rightside != null) {
    tweet = getTheDocumentUrlParameter("tweet");
    if (tweet == "" | | tweet ==null) {
   // do nothin and return a "" string ...
    }
    else {
       tweet = whole.substring(whole.indexOf('=') + 1,
       whole.length);
    }
 }
 return (tweet); }
 function GUI_configureElements() {
  workspace = document.getElementById('workspace');
   if (metro == true) canvasgroup = docu-
   ment.getElementById('canvasgroup') ;
  msi1 = document.getElementById('msi1');
  msi2 = document.getElementById('msi2');
  msi1.width = window.innerWidth;
  msi1.height = window.innerHeight;
   if (metro == false) {
       msi2.width = window.innerWidth;
       msi2.height = window.innerHeight;
   }
   context = msil.getContext("2d"); }
   function GUI_setDefaultBars() {
  ViewingAppBar = document.getElementById("ViewingAppBar");
  DrawingAppBar = document.getElementById("DrawingAppBar");
  hideBox("DrawingAppBar") ;
   if (_imaging_mode == "3D")
   {
       showBox("ViewingAppBar"); // only in 3dViewing (not
       in 2dViewing) !!
       showBox("PerfCounter");// for 2d only
   }
   else { // _imaging_mode == "2D" } }
   function GUI_setDefaults_2d() {
  _imaging_mode = "2D";
    GUI_setDefaultBars();
   if (metro == false) getUrlTweetParameter();
   if (tweet != "") {
   tweetUrl = tweet;
   }
   else {
   tweetUrl =
   "http://singapore.blob.core.windows.net/uploads/keyIma_2012-
   02-06T19:54:35-1f93d297-cb20-4ee6-bb58-fe49a6f2867f.jpg";
   }
  OnNavigatedToCanvas(tweetUrl); // for 2dVieing and
  3dViewing
  }
  function GUI_setDefaults_3d() {
  _imaging_mode = "3D";
    GUI_setDefaultBars();
   if (metro == false) getUrlTweetParameter();
   if (tweet != "") {
   tweetUrl = tweet;
   }
   else {
       tweetUrl =
       "http://singapore.blob.core.windows.net/config/singapore_cont
       extfolder_vol_1.tweet";
   }
  ViewingPage(this);
  OnNavigatedTo(tweetUrl); // 3dViewing
  }
  function GUI_setDefaults() {
  _imaging_mode = "2D";
    GUI_setDefaultBars();
   if (metro == false) getUrlTweetParameter();
   if (tweet != "") {
   tweetUrl = tweet;
   }
   else {
   tweetUrl =
   "http://singapore.blob.core.windows.net/uploads/keyIma_2012-
   02-06T19:54:35-1f93d297-cb20-4ee6-bb58-fe49a6f2867f.jpg";
   }
  ViewingPage(this); // for 3dViewing only
  OnNavigatedToCanvas(tweetUrl); // for 2dVieing and
  3dViewing
  }
  function GUI_setDefaults_old() {
  _imaging_mode = "2D";
    GUI_setDefaultBars();
   if (metro == false) getUrlTweetParameter();
   if (tweet != "") {
   tweetUrl = tweet;
   }
   else {
   // 3d loading
         tweetUrl =
         "http://singapore.blob.core.windows.net/config/singapore_cont
         extfolder_vol_1.tweet";
         fe49a6f2867f.jpg";
   }
  ViewingPage(this); OnNavigatedTo(tweetUrl); //
  3dViewing
  }
  function GUI_setEvents() {
  GUI_initWinRTPointerEventHandlersOnImage(); {
  function onWindowResize(event) {
   if (context != null) context.save();
   if (contextDrawing != null) contextDrawing.save();
  msi1.width = window.innerWidth;
  msi1.height = window.innerHeight;
  msi2.width = window.innerWidth;
  msi2.height = window.innerHeight;
   if (context != null) context.restore();
   if (contextDrawing != null) contextDrawing.restore();
   copy_img_to_canvas(); }
   function onDOMContentLoaded() {
   if (metro == true) gestureObject = new MSGesture();
  window.addEventListener("resize", onWindowResize, false);
  GUI_configureElements();
  GUI_setEvents();
  GUI_configureSketchupElements();
  GUI_setSketchupDefaults()
  GUI_setDefaults();
  }
  function GUI_ApplicationBarIconButton_ROTATE_Click() {
  ApplicationBarIconButton_ROTATE_Click(); }
  function GUI_ApplicationBarIconButton_WINDOWING_Click() {
  ApplicationBarIconButton_WINDOWING_Click(); }
  function GUI_ApplicationBarIconButton_START_Click() {
  ApplicationBarIconButton_START_Click(); }
  function GUI_ApplicationBarIconButton_2D_Click() {
  ApplicationBarIconButton 2D Click(); }
  function GUI_ApplicationBarIconButton_SaveKeyIma_Click() {
  }
  function GUI_initWinRTPointerEventHandlersOnImage()
  {
  GUI_onPointerEventsReset();
   if (metro == true) {
       msi = msi1;// set event-canvas to general msi varia-
       ble
       gestureObject.target = msi;
       msi.gestureObject = gestureObject;
       msi.addEventListener('MSPointerDown',
       GUI_onPointerDown, false);
       msi.addEventListener('MSPointerMove',
       GUI_onPointerMove, false);
       msi.addEventListener('MSPointerUp', GUI_onPointerUp,
       false) ;
       msi.addEventListener('MSPointerOut',
       GUI_onPointerOut, false);
       msi.addEventListener('MSDoubleTapped',
       GUI_onDoubleTapped, false); // kommt nicht
       msi.addEventListener("dblclick",
       GUI_onMouseDoubleTapped, false); // ok!
   }
   else {
       msi = msi1;
       msi1.addEventListener("mousedown", GUI_onMouseDown,
       false) ;
       //msi1.addEventListener("mousemove", GUI_onMouseMove,
       false) ;
       //msi1.addEventListener("mouseup", GUI_onMouseUp,
       false) ;
       //msi1.addEventListener("mouseout", GUI_onMouseOut,
       false) ;
       msi1.addEventListener("contextmenu",
       GUI_onContextMenu, false);
       msi1.addEventListener("onclick", GUI_onClick, false);
       msil.addEventListener("dblclick",
       GUI_onMouseDoubleTapped, false);
   }
   }
   function GUI_onPointerEventsReset() {
   if (viewing_on == false) return;// sketchup extension !!
   firstPointerId = 0;
  numActiveContacts = 0;
   contacts = null;
   contacts = new Object(); }
   function GUI_onDoubleTapped(/* PointerEventArgs */ e) {
   if (viewing_on == false) return;// sketchup extension !!
   //var pt = e.currentPoint.rawPosition;
  var pt = e;
  var ptrId = e.pointerId;
  ApplicationBarIconButton_RELOAD_Click(pt);
   e.preventDefault();
   e.preventMouseEvent() ;
   e.preventManipulation(); }
   function GUI_onPointerOut(event) {
   if (viewing_on == false) return;// sketchup extension !!
  GUI_onPointerUp(event); }
  function GUI_onPointerDown(/* PointerEventArgs */ e) {
   if (viewing_on == false) return;// sketchup extension !!
   if (numActiveContacts >= SUPPORTEDCONTACTS) {
       return;
   }
   if (numActiveContacts == 0) {
       firstPointerId = 0;
       contacts = null;
       contacts = new Object(); }
  var pt = e;
  var ptrId = e.pointerId;
  numActiveContacts++;
   if (numActiveContacts == 1) {
       firstPointerId = ptrId; }
   if ((ptrId > 0) && (ptrId == firstPointerId)) {
       contacts[ptrId] = pt; //.Position;
       ApplicationEvent_onPointerEventRaised(pt, numActive-
       Contacts, "PointerDown");
   }
   e.preventDefault();
   e.preventMouseEvent() ;
   e.preventManipulation(); }
   function GUI_onPointerMove(/* PointerEventArgs */ e) {
   if (viewing_on == false) return;
  var pt = e;
  var ptrId = e.pointerId;
   if (numActiveContacts == 0) {
   e.preventDefault();
   e.preventMouseEvent() ;
   e.preventManipulation() ;
       return; // ignore moves after pointer up event
   }
   if ((ptrId > 0) && (ptrId == firstPointerId)) { // point-
   er-moves only for a single common pointer not for all active
   pointers because they are moving in sync in our case!
       ApplicationEvent_onPointerEventRaised(pt, numActive-
       Contacts, "PointerMove");
   }
   e.preventDefault();
   e.preventMouseEvent() ;
   e.preventManipulation(); }
   function GUI_onPointerUp(/* PointerEventArgs */ e) {
   if (viewing_on == false) return;
  var pt = e;
  var ptrId = e.pointerId;
   if (numActiveContacts <= 0) { // maybe up- and out-events
   coming in sequence, so only handle one of them as up-event!
   e.preventDefault();
   e.preventMouseEvent() ;
   e.preventManipulation() ;
       return;
   }
   if ((ptrId > 0) && (ptrId == firstPointerId)) { // we use
   the total number of contacts used in generating this event
   for the selection of left(1), middle(2) or right(3)
       ApplicationEvent_onPointerEventRaised(pt, numActive-
       Contacts, "PointerUp");
   }
   if (numActiveContacts > 0) numActiveContacts--;
   if (numActiveContacts == 0) {
       firstPointerId = 0;
       contacts = null;
   }
   e.preventDefault();
   e.preventMouseEvent() ;
   e.preventManipulation(); }
   function GUI_onTouchDoubleTapped(/* PointerEventArgs */ e) {
   if (viewing_on == false) return;// sketchup extension !!
  var pt = new Point();
  pt.x = e.changedTouches.item(0).pageX;
  pt.y = e.changedTouches.item(0).pageY;
  ApplicationBarIconButton_RELOAD_Click(pt);
  }
  function GUI_onTouchOut(event) {
   if (viewing_on == false) return;// sketchup extension !!
  GUI_onTouchUp(event) ; }
  function GUI_onTouchDown(/* PointerEventArgs */ e) {
   if (viewing_on == false) return;// sketchup extension !!
   if (numActiveContacts >= SUPPORTEDCONTACTS) {
       return; // cannot support more contacts }
   if (numActiveContacts == 0) {
       firstPointerId = 0;
       contacts = null;
       contacts = new Object(); }
  var pt = new Point();
  pt.x = e.changedTouches.item(0).pageX;
  pt.y = e.changedTouches.item(0).pageY;
  var ptrId = e.touches.item(0).identifier;
  numActiveContacts = e.changedTouches.length;
   if (numActiveContacts == 1) {
       firstPointerId = ptrId;
   }
   contacts[ptrId] = pt; //.Position;
  ApplicationEvent_onPointerEventRaised(pt, numActiveCon-
  tacts, "PointerDown");
  }
  function GUI_onTouchMove(/* PointerEventArgs */ e) {
   if (viewing_on == false) return;// sketchup extension !!
  var pt = new Point();
  pt.x = e.changedTouches.item(0).pageX;
  pt.y = e.changedTouches.item(0).pageY;
  var ptrId = e.changedTouches.item(0).identifier;
   if (numActiveContacts == 0) {
       return; // ignore moves after pointer up event
   }
  ApplicationEvent_onPointerEventRaised(pt, numActiveCon-
  tacts, "PointerMove");
  }
  function GUI_onTouchUp(/* PointerEventArgs */ e) {
   if (viewing_on == false) return;// sketchup extension !!
  var pt = new Point();
  pt.x = e.changedTouches.item(0).pageX;
  pt.y = e.changedTouches.item(0).pageY;
  var ptrId = e.changedTouches.item(0).identifier;
   if (numActiveContacts <= 0) { // maybe up- and out-events
   coming in sequence, so only handle one of them as up-event!
       return;
   }
  ApplicationEvent_onPointerEventRaised(pt, numActiveCon-
  tacts, "PointerUp");
   firstPointerId = 0;
   contacts = null; }
   function GUI_Webkit_onTouchDoubleTapped(/* PointerEventArgs
   */ e) {
   if (viewing_on == false) return;// sketchup extension !!
  var pt = new Point();
  pt.x = e.changedTouches.item(0).pageX;
  pt.y = e.changedTouches.item(0).pageY;
  ApplicationBarIconButton_RELOAD_Click(pt);
   e.preventDefault(); }
   function GUI_Webkit_onTouchOut(event) {
   if (viewing_on == false) return;// sketchup extension !!
  GUI_Webkit_onTouchUp(event); }
  function GUI_Webkit_onTouchDown(/* PointerEventArgs */ e) {
   if (viewing_on == false) return;// sketchup extension !!
  var pt = new Point();
  pt.x = e.changedTouches.item(0).pageX;
  pt.y = e.changedTouches.item(0).pageY;
  var ptrId = e.changedTouches.item(0).identifier;
  numActiveContacts = e.touches.length;
  ApplicationEvent_onPointerEventRaised(pt, numActiveCon-
  tacts, "PointerDown");
   e.preventDefault(); }
   function GUI_Webkit_onTouchMove(/* PointerEventArgs */ e) {
   if (viewing_on == false) return;// sketchup extension !!
  var pt = new Point();
  pt.x = e.changedTouches.item(0).pageX;
  pt.y = e.changedTouches.item(0).pageY;
  var ptrId = e.changedTouches.item(0).identifier;
  ApplicationEvent_onPointerEventRaised(pt, numActiveCon-
  tacts, "PointerMove");
   e.preventDefault(); }
   function GUI_Webkit_onTouchUp(/* PointerEventArgs */ e) {
   if (viewing_on == false) return;// sketchup extension !!
  var pt = new Point();
  pt.x = e.changedTouches.item(0).pageX;
  pt.y = e.changedTouches.item(0).pageY;
  var ptrId = e.changedTouches.item(0).identifier;
  ApplicationEvent_onPointerEventRaised(pt, numActiveCon-
  tacts, "PointerUp");
   e.preventDefault(); }
   function GUI_onClick(/* PointerEventArgs */ e) {
   if (viewing_on == false) return;// sketchup extension !!
   if (metro == true) e.preventDefault();
   if (e && e.stopPropagation)
     e.stopPropagation();
   return false; }
   function GUI_onContextMenu(/* PointerEventArgs */ e) {
   if (viewing_on == false) return;// sketchup extension !!
   if (metro == true) e.preventDefault();
   if (e && e.stopPropagation)
     e.stopPropagation();
   return false; }
   function GUI_onMouseDoubleTapped(/* PointerEventArgs */ e) {
   if (viewing_on == false) return;// sketchup extension !!
  var pt = new Point();
  pt.x = e.clientX;
  pt.y = e.clientY;
  ApplicationBarIconButton_RELOAD_Click(pt); }
  function GUI_onMouseOut(event) {
   if (viewing_on == false) return;// sketchup extension !!
  GUI_onMouseUp(event); }
  function GUI_onMouseDown(/* PointerEventArgs */ e) {
   if (viewing on == false) return;// sketchup extension !!
   if (metro == true) e.preventDefault();
  msi.addEventListener("mousemove", GUI_onMouseMove,
  false) ;
  msi.addEventListener("mouseup", GUI_onMouseUp, false);
  msi.addEventListener("mouseout", GUI_onMouseOut, false);
  var pt = new Point();
  pt.x = e.clientX;
  pt.y = e.clientY;
  var ptrId = e.button + 1;
  numActiveContacts = e.button + 1;
  ApplicationEvent_onPointerEventRaised(pt, numActiveCon-
  tacts, "PointerDown");
  Dragging = true; }
  function GUI_onMouseMove(/* PointerEventArgs */ e) {
   if (viewing_on == false) return;// sketchup extension !!
   if (metro == true) e.preventDefault();
  var pt = new Point();
  pt.x = e.clientX;
  pt.y = e.clientY;
  var ptrId = e.button + 1;
   if (Dragging == true)
     ApplicationEvent_onPointerEventRaised(pt, numActive-
     Contacts, "PointerMove");
     }
     function GUI_onMouseUp(/* PointerEventArgs */ e) {
   if (viewing_on == false) return;// sketchup extension !!
   if (metro == true) e.preventDefault();
  var pt = new Point();
  pt.x = e.clientX;
  pt.y = e.clientY;
  var ptrId = e.button + 1;
  numActiveContacts = e.button + 1;
  ApplicationEvent_onPointerEventRaised(pt, numActiveCon-
  tacts, "PointerUp");
  msi.removeEventListener("mousemove", GUI_onMouseMove,
  false) ;
  Dragging = false; }
  function GUI_set_2D_Image_Visibility(visibility) {
   if (visibility == true) showBox("container"); //
   $("#container").css({ opacity: 1.0 });
   else hideBox("container"); // $("#container").css({
   opacity: 0.0 });
   }
   function GUI_set_3D_Image_Visibility(visibility) {
   if (visibility == true) showBox("Imagel"); // $("#Im-
   agel").css({ opacity: 1.0 });
   else hideBox("Image1"); // $("#Imagel").css({ opacity:
   0.0 });
   }
   function GUI_set_Video_Image_Visibility(visibility) {
   }
   function GUI_set_3D_Image_Source(result, uriCalled, nr) {
   }
   function GUI_set_RBDM_Content(val) {
  windowing = document.getElementById("windowingButton");
   if (val == "D") {
       if (metro == true) {
           windowing.winControl.label = 'dragging';
           windowing.winControl.icon = 'D';
           windowing.winControl.tooltip = 'dragging';
       } else {
           windowing.src = "imag-
           es/ApplicationBar.Dragging.png";
       }
   }
   else {
       if (metro == true) {
           windowing.winControl.label = 'windowing';
           windowing.winControl.icon = 'W';
           windowing.winControl.tooltip = 'windowing'; }
       else {
           windowing.src = "imag-
           es/ApplicationBar.Windowing.png";
           }}}
           function GUI_set_LBDM_Content(val) {
           }
           function GUI_set_RZP_Content(val) {
   rotateButton = document.getElementById("rotateButton");
   if (val == "Z") {
       if (metro == true) {
           rotateButton.winControl.label = 'zoom';
           rotateButton.winControl.icon = 'Z';
           rotateButton.winControl.tooltip = 'zoom'; }
       else {
           rotateButton.src = "imag-
           es/ApplicationBar.Zoom.png";
       }
   }
   if (val == "R") {
       if (metro == true) {
           rotateButton.winControl.label = 'rotate';
           rotateButton.winControl.icon = 'R';
           rotateButton.winControl.tooltip = 'rotate';
       else {
           rotateButton.src = "imag-
           es/ApplicationBar.Rotate.png";
       }
   }
   if (val == "P") {
       if (metro == true) {
       rotateButton.winControl.label = 'pan';
       rotateButton.winControl.icon = 'P';
       rotateButton.winControl.tooltip = 'pan';
       }
       else {
           rotateButton.src = "imag-
           es/ApplicationBar.Pan.png";
           }}}
           function GUI_set_StartButton_Content(val) {
   startButton = document.getElementById("startButton");
   if (val == "Start") {
       if (metro == true) {
           startButton.winControl.label = val;
           startButton.winControl.icon = 'play'; // 0xE102;
           // ''
       }startButton.winControl.tooltip = 'start';
       else {
           startButton.src = "imag-
           es/ApplicationBar.Start.png";
       }
   }
   else {
       if (metro == true) {
           startButton.winControl.label = val;
           startButton.winControl.icon = 'stop'; // 0xE102;
           // ''
           startButton.winControl.tooltip = 'stop';
       }
       else {
           startButton.src = "imag-
           es/ApplicationBar.Stop.png";
           }}}
           function GUI_set_ModeButton_Content(val) {
   zweiDButton = document.getElementById("zweiDButton");
   if (val == "2D") {
       if (metro == true) {
           zweiDButton.winControl.label = '2D';
           zweiDButton.winControl.icon = '2';
           zweiDButton.winControl.tooltip = '2D';
       }
       else {
       }zweiDButton.src = "images/ApplicationBar.2D.png";
   }
   else {
       if (metro == true) {
           zweiDButton.winControl.label = '3D';
           zweiDButton.winControl.icon = '3';
           zweiDButton.winControl.tooltip = '3D';
       }
       else {
           zweiDButton.src = "images/ApplicationBar.3D.png";
           }}}
           function GUI_set_ListTitle_Text(val) {
           }
           function GUI_set_RBDMButton_Visibility(visibility) {
  document.getElementById("windowingButton").Visibility =
  visibility; // RBDMButton
  }
  function GUI_set_LBDMButton_Visibility(visibility) {
  }
  function GUI_set_RZPButton_Visibility(visibility) {
  document.getElementById("rotateButton").Visibility = vis-
  ibility;
  }
  function GUI_set_StartButton_Visibility(visibility) {
  document.getElementById("startButton").Visibility = visi-
  bility;
  }
  function GUI_set_ModeButton_Visibility(visibility) {
  document.getElementById("zweiDButton").Visibility = visi-
  bility;
  }
  function GUI_display_blob(blob) {
  document.getElementById("msi2").src = blob; // ok, if
  <img > used
  }
  function GUI_display_blob_base64 (blob) {
  document.getElementById("msi2").src = 'da-
  ta:image/jpeg;base64,' + blob; // ok, if <img > used
  }
  function GUI_display_fps(avgDelay) {
   fps = document.getElementById("fps");
   }fps.innerHTML = (1000/avgDelay).toFixed(1) + " fps";
   function GUI_configureSketchupElements() {
 contextDrawing = null;
 workspaceDrawing = document.getElementById('workspace');
 document.getElementById('workspaceDrawing');
 if (workspaceDrawing != null) {
  msi1 = document.getElementById('msi1');
   canvas = msi1; // changed because same canvas is used for
   drawing and viewing!!
   contextDrawing = canvas.getContext("2d");
   }}viewing_on = true;
   function GUI_setSketchupDefaults() {
   if (contextDrawing != null) {
       currentTool = pencil = new tool.pencil();
       contextDrawing.lineWidth = 4;
       contextDrawing.strokeStyle = '#A6A6A6'; // White col-
       or
       contextDrawing.fillStyle = '#FFF';
       contextDrawing.fillRect = (0, 0, canvas.width, can-
       vas.height) ;
       }}
       function showBox(boxid) { // make visible e.g. <div id = xxx>
  document.getElementById(boxid).style.visibility = "visi-
  ble";
  document.getElementById(boxid).disabled = false;
  }
  function hideBox(boxid) { // make invisible e.g. <div id =
  xxx>
  document.getElementById(boxid).style.visibility = "hid-
  den";
  document.getElementById(boxid).disabled = true;
  }
  function onNewSketch(event) {
   contextDrawing.clearRect = (0, 0, canvas.width, can-
   vas.height);
   setDefaults(); }
   function startCanvasEvents() {
   if (metro == true) {
   canvas.addEventListener("MSPointerDown", onCanvasDown,
   false) ;
   canvas.addEventListener("MSPointerMove", onCanvasMove,
   false) ;
   canvas.addEventListener("MSPointerUp", onCanvasUp,
   false) ;
   canvas.addEventListener("MSPointerOut", onCanvasOut,
   false) ;
   }
   else {
   canvas.addEventListener("mousedown", onCanvasDown,
   false) ;
   //canvas.addEventListener("mousemove", onCanvasMove,
   false) ;
   //canvas.addEventListener("mouseup", onCanvasUp, false);
   //canvas.addEventListener("mouseout", onCanvasOut,
   false) ;
   }}
   function stopCanvasEvents() {
   if (metro == true) {
   canvas.removeEventListener("MSPointerDown", onCan-
   vasDown, false);
   canvas.removeEventListener("MSPointerMove", onCan-
   vasMove, false);
   canvas.removeEventListener("MSPointerUp", onCanvasUp,
   false) ;
   canvas.removeEventListener("MSPointerOut", onCanvasOut,
   false) ;
   }
   else {
   canvas.removeEventListener("mousedown", onCanvasDown,
   false) ;
   canvas.removeEventListener("mousemove", onCanvasMove,
   false) ;
   canvas.removeEventListener("mouseup", onCanvasUp,
   false) ;
   canvas.removeEventListener("mouseout", onCanvasOut,
   false) ;
   } }
   function onCanvasDown(event) {
   canvas.addEventListener("mousemove", onCanvasMove,
   false) ;
   canvas.addEventListener("mouseup", onCanvasUp, false);
   canvas.addEventListener("mouseout", onCanvasOut, false);
  var source = event.currentTarget;
   currentPos = getxy(canvas, event);
   if (currentTool.name != 'select' && currentTool.name !=
   'eraser') {
       if (event.button == 2 && currentTool.name != 'eras-
       er') {
           var temp = contextDrawing.strokeStyle;
           contextDrawing.strokeStyle = contextDraw-
           ing.fillStyle;
           contextDrawing.fillStyle = temp; }
   }
   currentTool.down(event) ;
   currentTool.status = 1; }
   function onCanvasUp(event) {
   currentPos = getxy(canvas, event);
   event.stopPropagation() ;
   currentTool.up(event) ;
   currentTool.status = 0;
   canvas.removeEventListener("mousemove", onCanvasMove,
   false) ;
   return false; }
   function onCanvasMove(event) {
   currentPos = getxy(canvas, event);
   event.stopPropagation();
   if (currentTool.status > 0) {
       currentTool.move(event);
   }
   return false; }
   function onCanvasOut(event) {
   if (contextDrawing && (currentTool.name == 'pencil' | |
   currentTool.name == 'eraser' | |
       currentTool.name == 'brush') && currentTool.status ==
       1) {
       currentTool.disconnected = 1;
       currentTool.draw();
       } }
       var tool = {
  pencil: function () {
       this.name = 'pencil';
       this.status = 0;
       this.inherit = tool._brushes; this.inherit();
   }, contextDrawing.lineCap = 'butt';
   eraser: function () {
       this.name = 'eraser';
       this.status = 0;
       this.inherit = tool._brushes; this.inherit();
   }, contextDrawing.lineCap = 'square';
  _brushes: function () {
       this.down = function () {
           if (currentTool.status == 0) {
               this.start = { x: currentPos.x, y: current-
               Pos.y }
               currentTool.status = 1;
           } else {
               this.draw() ;
           contextDrawing.moveTo(currentPos.x, current-
           Pos.y); }
       this.up = function () {
           if (this.start && this.start.x == currentPos.x &&
           this.start.y == currentPos.y) {
               // drawDot(currentPos.x, currentPos.y, con-
               text.lineWidth, context.strokeStyle, context);
           }
           this.start = null;
           currentTool.status = 0;
       }
       this.move = function () {
           this.last = { x: currentPos.x, y: currentPos.y }
           this.draw();
           contextDrawing.moveTo(currentPos.x, current-
           Pos.y); }
       this.draw = function () {
           contextDrawing.1ineTo(currentPos.x, current-
           Pos.y);
           contextDrawing.stroke() ;
           contextDrawing.beginPath();
           this.last = { x: currentPos.x, y: currentPos.y }
           } } }
           function drawDot(x, y, size, color, trg) {
  x = Math.floor(x) + 1;
   y = Math.floor(y) + 1;
   if (x > 0 && y > 0) {
       if (!trg) {
       }trg = contextDrawing;
       if (color | | size) {
           var lastcol = trg.fillStyle;
       }var lastsize = trg.lineWidth;
       if (color) {
           trg.fillStyle = color;
       }
       if (size) {
           trg.lineWidth = size; }
       var dotoffset = (trg.lineWidth > 1) ? (trg.lineWidth
       / 2) : 0;
       trg.fillRect((x - dotoffset), (y - dotoffset),
       trg.lineWidth, trg.lineWidth);
       if (color | | size) {
           trg.fillStyle = lastcol;
           trg.lineWidth = lastsize;
           } } }
           function getxy(object, event) {
   if (contextDrawing) {
       var bo = getPosition(object); // Attention: msi is
       canvas
       var x = event.clientX - bo.x + workspaceDraw-
       ing.scrollLeft;
       var y = event.clientY - bo.y + workspaceDraw-
       ing.scrollTop;
       x += document.documentElement.scrollLeft;
       y += document.documentElement.scrollTop;
       x = (contextDrawing.zoom) ? x / contextDrawing.zoom :
       x;
       y = (contextDrawing.zoom) ? y / contextDrawing.zoom :
       y;
       return { x: x - .5, y: y - .5 }; // prevent anti-
       aliasing
   }
   else {
       var x = 0;
       var y = 0;
   }return { x: x, y: y };
   }
   function getPosition(object) {
  var bo, x, y, b;
  x = y = 0;
  bo = object.getBoundingClientRect();
  x = bo.left;
   y = bo.top;
   }return { x: x, y: y }
   function setColor(red, green, blue) {
  var rgb = "rgb(" + red + ", " + green + ", " + blue +
  ")" ;selectColor(null, null, rgb); }
  function selectColor(object, event, rgb) {
   color = rgb ? rgb : object.style.backgroundColor;
   currentColor = color;
   contextDrawing.strokeStyle = color;
   if (contextDrawing.lastStrokeStyle) {
       contextDrawing.lastStrokeStyle = color; }
       }
       function setPen(size) {
   contextDrawing.lineWidth = size; }
   function clearCanvas() {
   contextDrawing.clearRect(0, 0, canvas.width, can-
   vas.height) ;
   }
   function msil_Loaded() {
   }
   function msi1_Unloaded() {
   }
   function msi1_ImageFailed() {
   }
   function copy_img_to_canvas() {
   if (viewing_on == false) {
   var blob1 = viewer.drawer.elmt.firstChild;
       var context1 = canvas.getContext("2d")
   if (metro == true) {
         context1.drawImage(blob1, blob1.offsetLeft,
         blob1.offsetTop, blob1.offsetHeight, blob1.offsetWidth);
    }
   else {
         context1.drawImage(blob1, 0,0);
         } } }
         function GUI_SwitchAppBar_Click() {
   switchAppBarButton = docu-
   ment.getElementById("switchAppBarButton") ;
   if (viewing_on == true) {
       hideBox("ViewingAppBar") ;
       showBox("DrawingAppBar") ;
       startCanvasEvents();
       viewing_on = false;
   var blob1;
   if (_imaging_mode == "3D") {
        blob1 = document.getElementById("msi2"); }
   else {
         if (mode == "3D") {
              blob1 = viewer.drawer.elmt.firstChild;
         }
         else {
         hideBox ("container");
                   blob1 = document.getElementById("msi2"); } }
         var context1 = canvas.getContext("2d")
   if (metro == true) {
         context1.drawImage(blob1, blob1.offsetLeft,
         blob1.offsetTop, blob1.offsetHeight, blob1.offsetWidth);
    }
   else {
         if (_imaging_mode == "3D")
              context1.drawImage(blob1, 0,0, win-
              dow.innerWidth,window.innerHeight) ;
         else
              context1.drawImage(blob1, 0,0); }
   if (metro == true) {
         switchAppBarButton.winControl.label = 'viewing';
         switchAppBarButton.winControl.icon = 'view'; //
         0xE102; // ''
         switchAppBarButton.winControl.tooltip = 'viewing'; }
   else {
         switchAppBar-
         Button.src="images/ApplicationBar.Refresh.png";
    }
   }
   else {
   if (mode == "3D") {
        blob1 = viewer.drawer.elmt.firstChild; // vp-
        firstchild of drawer = canvas
    }
   else {
              blob1 = document.getElementById("msi2"); }
       contextDrawing.clearRect(0, 0, canvas.width, can-
       vas.height);
       stopCanvasEvents();
       viewing_on = true;
       hideBox("DrawingAppBar") ;
   if (_imaging_mode == "3D" | | mode == "2D") {
         showBox("ViewingAppBar"); // only in 3dViewing !! }
   if (_imaging_mode == "2D" && mode == "2D") {
         if (msil_Opacity == 100 && _loop_mode == "2DCine"
         && _config_content_type == "volume" && _MSI_3d_2d_loaded) {
         }
         else {
              showBox("container"); }
    }
   if (metro == true) {
         switchAppBarButton.winControl.label = 'drawing';
         switchAppBarButton.winControl.icon = 'highlight';
         // 0xE102; // ''
         switchAppBarButton.winControl.tooltip = 'drawing'; }
   else {
         switchAppBar-
         Button.src="images/ApplicationBar.Edit.png";
    }
   }
   function GUI _ViewDetail_Click() {
   if (_imaging_mode == "2D") {
         if (viewing_on == false) {
              GUI_SwitchAppBar_Click(); }
        _imaging_mode = "3D";
         GUI_setDefaultBars();
         set_ModeButton_Content("3D");
        ApplicationBarIconButton_2D_Click();
         return; }
  viewingdetailButton = docu-
  ment.getElementById("viewingdetailButton") ;
   if (zoomin == true) {
   if (metro == true) {
         viewingdetailButton.winControl.label = 'faster';
         viewingdetailButton.winControl.icon = 'zoomout';
         viewingdetailButton.winControl.tooltip = 'faster'; }
   }
   else {
   if (metro == true) {
         viewingdetailButton.winControl.label = 'details';
         viewingdetailButton.winControl.icon = 'zoomin';
         viewingdetailButton.winControl.tooltip = 'details';
         } } }
         function GUI_import_Click() {
 if (metro == true) {
  var img = new Image();
   img.onload = function () {
       setTimeout(function () {
           var scaleFactor = Math.max(window.innerHeight /
           img.naturalHeight, window.innerWidth / img.naturalWidth);
           contextDrawing.drawImage(img, 0, 0,
           img.naturalWidth, img.naturalHeight, 0, 0, canvas.width, can-
           vas.width);
       }, 100);
       currentImage = img;
   };
  var picker = new Win-
  dows.Storage.Pickers.FileOpenPicker();
  picker.fileTypeFilter.replaceAll([".jpg", ".bmp", ".gif",
  ".png"]);
  picker.pickSingleFileAsync().then(function (file) {
       var blobUrl = URL.createObjectURL(file);
       img.src = blobUrl;
       currentBlobUrl = blobUrl;
   });
   } }
   function GUI_export_Click() {
 if (metro == true) {
  var blob = canvas.msToBlob();
  var picker = new Win-
  dows.Storage.Pickers.FileSavePicker();
  picker.viewMode = Win-
  dows.Storage.Pickers.PickerViewMode.thumbnail;
  picker.fileTypeChoices.insert("PNG", [".png"]);
  picker.pickSaveFileAsync().then(function (file) {
       if (file != null) {
       file.openAsync(Windows.Storage.FileAccessMode.readWrite) .then
       (function (output) {
               // Get the IInputStream stream from the blob
               object
               var input = blob.msDetachStream();
               // Copy the stream from the blob to the File
               stream
               Win-
               dows.Storage.Streams.RandomAccessStream.copyAsync(input, out-
               put) .then(function () {
                   output.flushAsync().then(function () {
                       input.close() ;
                       output.close() ;
                       //sdkSample.displayStatus("File '" +
                       file.name + "' saved successfully to the Pictures Library!");
                     });
                 });
             }); }
   });
   } }
   function isMobileDevice() {
   if( navigator.userAgent.match(/Android/i)
   | | navigator.userAgent.match(/webOS/i)
   | | navigator.userAgent.match(/iPhone/i)
   | | navigator.userAgent.match(/iPad/i)
   | | navigator.userAgent.match(/iPod/i)
   | | navigator.userAgent.match(/BlackBerry/i)
   | | navigator.userAgent.match(/Windows Phone/i)
   ) {
   return true;
 }
 else {
   return false;
   } }
   function checkJpgfileFromUri(uri) {
  var uriPath = uri.substring(0, uri.lastIndexOf("/") + 1);
  // e.g.
  "http://singapore.blob.core.windows.net/mysingapore/Generated
  ImagesDLR/"
  var xhr = new XMLHttpRequest();
  xhr.open('GET', uri, true); // firefox, chrome, safari
  xhr.responseType = 'image/jpeg'; // ok fuer firefox,
  chrome, safari and IE9
  xhr.onreadystatechange = function () {
       if (xhr.readyState == 4) {
           if (xhr.status == 200) {
                 DziImageFormat = ".jpg"; }
           else {
               DziImageFormat = ".png"; // kd Attention: has
               to be improved finally (could be network problem or other
               file type like tiff as well).
           }
            initViewer(tweetUrl); // this fills the thumb
            bar with collection overview images
            var colllndex = 0;
            loadDzcImage(collIndex)
       }
   };
  xhr.send(null); }
  function loadDZCfileFromUri(uri) {
  var uriPath = uri.substring(0, uri.lastIndexOf("/") + 1);
  // e.g.
  "http://singapore.blob.core.windows.net/mysingapore/Generated
  ImagesDLR/"
  var xhr = new XMLHttpRequest();
  xhr.open('GET', uri, true); // firefox, chrome, safari
  xhr.responseType = 'text'; // ok fuer firefox, chrome,
  safari and IE9
  xhr.onreadystatechange = function () {
       if (xhr.readyState == 4) {
           if (xhr.status == 200) {
               var isColl = parseDZCdata(this.responseText,
               uriPath);
                 if (isColl == false) {
                      // in this case the tweet is a DZI-
                      file and not a DZC-file (missing collection tag) and so we
                      have a single dzi fle and need no parsing. we have not to
                      wait for file load finished
                      srcFileToLoadType = "dzi";
                      srcFileToLoad = tweetUrl;
                      initViewer(tweetUrl);
                      loadDziImage(srcFileToLoad);
                      return;
                 }
                 var firstiref = collectionDZIImaRef[0];
               myUri = firstiref + "_files/" + tileLevel-
               ForStamp + "/0_0.jpg"; // !!!!! kd 19.1.2013 Achtung: hier
               muss man auch .png nehmen koennen!!!!!!
                 DziImageFormat = ".png";
                 // only if the following call succeeds the
                 imageFormat can be changed to .jpg !
                 checkJpgfileFromUri(myUri);
                 return;
           }
           else {
               alert(' error imgLoaderURL: ' +
               xhr.statusText);
           }
       }
   };
  xhr.send(null); }
  function parseDZCdata(xmlString, uriPath) {
  var newContextLine = ""
  xmlns=\"http://schemas.datacontract.org/2004/07/viewingAppXam
  1\">';
  var corrString = correctXmlString(xmlString /*lines*/,
  newContextLine, false);
  var xmldoc = $.parseXML(corrString);
  var allitems = xmldoc.getElementsByTagName("Collection");
   if (allitems.length <= 0) {
       // return false if we do not have a collection. Maybe
       we have a dzi-file with .xml extension
        return (false);
   }
  var allContextNodes = $(xmldoc);
   collectionDZIImaRef = 0;
   collectionDZIImaRef = new Array();
   $(xmldoc).find("I").each(function()
   {
   var item = $(this).attr("Source"); // e.g.
   "dzc_output_images/41214375.xml"
        var itemRef = item.substring(0,
        item.lastlndex0f(".") + 0); // "dzc-output_images/41214375"
        var itemXmlFile = itemRef + ".xml"; // not used di-
        rectly here, but shows the way how to get to dzi-xml file
        var itemOverviewJpgFile = itemRef +
        "_files/8/0_0.jpg"; // not used directly here, but shows the
        way how to get to dzi-overview-jpg file for thumb bar if
        needed
        var itemOverviewPngFile = itemRef +
        "_files/8/0_0.png"; // not used directly here, but shows the
        way how to get to dzi-overview-png file for thumb bar if
        needed
        collectionDZIImaRef.push(uriPath + itemRef); // e.g.
        "http://singapore.blob.core.windows.net/mysingapore/Generated
        ImagesDLR/dzc_output_images/41214375"
        collectionDZIImaAnz++;
   });
   return (true); }
   function loadContextFromUri(uri) {
  var xhr = new XMLHttpRequest();
  xhr.open('GET', uri, true);
  xhr.responseType = 'text';
  xhr.onreadystatechange = function () {
       if (xhr.readyState == 4) {
           if (xhr.status == 200) {
               parseContextData(this.responseText); }
           else {
               //alert(' error imgLoaderURL: ' +
               xhr.statusText);
           }
       }
   };
  xhr.send(null); }
  function parseContextData(xmlString) {
  var newContextLine = ""; // '<Context
  xmlns:i=\"http://www.w3.org/2001/XMLSchema-instance\"
  xmlns=\"http://schemas.datacontract.org/2004/07/viewingAppXam
  1\">';
  var corrString = correctXmlString(xmlString /*lines*/,
  newContextLine, true);
  var xmldoc = $.parseXML(corrString);
  var allContextNodes = $(xmldoc);
  var g = (G() + G() + "-" + G() + "-" + G() + "-" + G() +
  "-" + G() + G() + G()).toUpperCase(); // Sys-
  tem.Guid.NewGuid().ToString();
  var i = new Instancelnfo(g);
   i.State = "Initial";
  var myServerBaseUri = // c.CloudTableBaseUri +
                                                "Cloud=" +
                                                $(allContextNodes).find("
CloudBlobBaseUri").text() +
                                                "TenantSto-
                                                rage=" + $(allCon-
                                                textNodes).find("CloudStorageAccount").text() +
                       // c.CloudComputeBaseUri +
                                                "TenantCom-
                                                pute=" + $(allCon-
                                                textNodes).find(
"CloudStorageAccount").text() +
                                                "Procedure="
                                                + $(allContextNodes).
find("ProcedureUid").text() +
                                                "AppHost=" +
                                                $(allContextNodes).find("AppHost").text() +
                                                "ConfigHost="
                                                + $(allContextNodes).find("ConfigHost").text() +
                                                "Com-
                                                puteHost=" + $(allConte
xtNodes).find("ComputeHost").text() +
                                                "Stor-
                                                ageHost=" + $(allCont
extNodes).find("StorageHost").text() +
                                                "StorageHost-
                                                VolumeSeries2d=" + $(allCon-
                                                textNodes).find("St
orageHostVolumeSeries2d").text() +
                                                "StorageHost-
                                                VolumeSeries2dAnz=" + $(allCon-
                                                textNodes).find("Stora
geHostVolumeSeries2dAnz") .text();
   i.content = $(allContextNodes).find("Content").text();
   i.thumb = $(allContextNodes).find("Thumb").text();
   i.StorageType = $(allCon-
   textNodes).find("StorageType").text();
   i.StorageFormat = $(allCon-
   textNodes).find("StorageFormat").text();
   i.drive = $(allContextNodes).find("Drive").text();
   i.nameUC = $(allContextNodes).find("NameUC").text();
   i.ServerBaseUri = "";
   if ($(allContextNodes).find("Content").text() == "vol-
   ume") { // only volumes need special infos
       i.ServerBaseUri = myServerBaseUri; }
   else {
       i.ServerBaseUri = $(allCon-
       textNodes).find("ServerBaseUri").text(); // other use the
       normal ServerBaseUri from config
   }
   i.DZMetaDataRelPath = $(allCon-
   textNodes).find("DZMetaDataRelPath") .text();
   i.DZInstDataRelPath = $(allCon-
   textNodes).find("DZInstDataRelPath") .text();
   i.DZMHDDataRelPath = $(allCon-
   textNodes).find("DZMHDDataRelPath") .text();
   i.ARegion = $(allContextNodes).find("ARegion").text();
   i.Guid = g;
  myInstanceInfo = i;
   instanceList_Selection_restart(); }
   function correctXmlString(inpString /* lines */, newContext,
   headerAndTrailer) {
  var lines;
  var TA = inpString;
   lines = TA.split("\r\n");
  var xmlString = "";
  var xmlHeader = "<?xml version='1.0'?>";
  var xmlStringHeader = "<Contexts>";
  var xmlStringTrailer = "</Contexts>";
   if (headerAndTrailer) {
       //xmlString = xmlHeader + xmlStringHeader + inpString
       + xmlStringTrailer;
       xmlString += xmlHeader + "\r\n"; // + inpString;
   }
   else {
       // xmlString = inpString; }
  var line;
  var il = 0;
  var ol = 0;
   for (il = 0; il < lines.length; il++) {
       line = lines[il];
       var context = (line.search("<Context xmlns:i=") != -
       1);
       if (newContext == "") {
           newContextLine = "<Context>"; }
       if (context == true && newContext != null && newCon-
       textLine != "") {
           if (newContext != "") {
               xmlString += newContextLine + "\r\n"; //
               writer.WriteLine(newContextLine) ;
           }
           else {
               xmlString += "<Context>" + "\r\n"; // writ-
               er.WriteLine(newContextLine) ;
           }
       }
       else if (line.search("ArrayOf") != -1) {
           // do not insert such lines
       }
       else if (line.search("<PartitionKey") != -1) {
       }
       else if (line.search("<RowKey") != -1) {
       }
       else if (line.search("<Timestamp") != -1) {
       }
       else {
           xmlString += line + "\r\n";
           //writer.WriteLine(line) ;
       }
   }
  var finalXmlString = xmlString; //writer.ToString();
  }return (finalXmlString);
  function OnNavigatedToCanvas(tweetUrl) {
   if (isMobileDevice() == true) {
         smartPhoneDevice = true;
    }
   else {
         smartPhoneDevice = false; }
   state = 1;
   var par = tweetUrl;
   var extension = par.substring(par.lastIndexOf("."),
   par.length);
   var collFound =
   (par.toLowerCase().indexOf("dzc_output _images") > 0);
   if (par != "" && par != null) { // we have any tweet pa-
   rameter
         if ((extension == ".tweet" | | extension ==
         ".TWEET") && collFound == false) {
              srcFileToLoadType = "tweet";
              srcFileToLoad = "";
              loadContextFromUri(par); // this fills the
              myInstance structure after async loading completed in calling
              parseContextData. Then instanceList_Selection_restart is
              called from parseContextData!
              return;
         }
         else if ((extension == ".xml" | | extension ==
         ".XML") /* && collFound == true*/ ) {
              srcFileToLoadType = "xm1";
              srcFileToLoad = "";
              loadDZCfileFromUri(par); // this fills the
              collection after async loading completed. Then initViewer and
              loadDzcImage is called from the load-callback!
              return;
         }
         else if (extension == ".jpg" | | extension == ".JPG"
         | | extension == ".jpeg" | | extension == ".JPEG" | | extension
         == ".png" | | extension == ".PNG") {
              srcFileToLoadType = "jpg";
              srcFileToLoad = tweetUrl;
              initViewer(tweetUrl);
              loadJpgImage(srcFileToLoad) ;
              return; }
         else if ((extension == ".dzi" | | extension ==
         ".DZI") && collFound == false) {
              srcFileToLoadType = "dzi";
              srcFileToLoad = tweetUrl;
              initViewer(tweetUrl) ;
              loadDziImage(srcFileToLoad);
              return;
         } else {
              srcFileToLoadType = "jpg";
              srcFileToLoad = tweetUrl;
              initViewer(tweetUrl) ;
              loadJpgImage(srcFileToLoad) ;
              return;
    } }
   else { // we have no tweet parameter, so we set a de-
   fault jpg file!
         srcFileToLoadType = "jpg";
         srcFileToLoad =
         "http://singapore.blob.core.windows.net/uploads/keyIma_2012-
         02-06T19:54:35_1f93d297-cb20-4ee6-bb58-fe49a6f2867f.jpg";
         loadJpgImage(srcFileToLoad); } }
         function instanceList_Selection _restart() {
  var instanceInfo = myInstanceInfo; // in-
  stanceList.SelectedItem as InstanceInfo;
  REM InstanceLoad(instanceInfo.State, instanceInfo.Guid,
  instanceInfo.StorageType, instanceInfo.StorageFormat, in-
  stancelnfo.drive, instanceInfo.nameUC, instanceIn-
  fo.DZMHDDataRelPath, instanceInfo.ServerBaseUri, instanceln-
  fo.DZMetaDataRelPath, instanceInfo.DZInstDataRelPath, in-
  stanceInfo.content) ;
  }
  function REM_InstanceLoad(mState, mGuid, mStorageType, mStor-
  ageFormat, mdrive, mnameUC, mDZMHDDataRelPath, mServer-
  BaseUri, mDZMetaDataRelPath, mDZInstDataRelPath, mContent) {
  _config_content_type = mContent;
  _mContent = mContent;
   if (mContent == "video") // for videos ...
   {
       set_ListTitle_Text(" media");
       set_ModeButton_Visibility("Collapsed"); // ModeBut-
       ton.Visibility = Visibility.Collapsed;
       _imaging_mode = "2D"; // current will be 3d
       now
       set_ModeButton_Content("2D"); // ModeButton.Content =
       "2D";
       _loop_mode = "video"; // current will be 3d
       eine
       set_3D_Image_Visibility(false); // msi1.Opacity = 0;
       set_2D_Image_Visibility(false); // msi.Opacity = 0;
       Menue3dPressed = false;
   }
   else if (mContent == "volume") // for volumes & 2d-Cine &
   2d/3d from this volume config entry (e.g. DZ files) ...
   {
       var x = mServerBaseUri.replace(/=/g, "|"); // JavaS-
       cript supports only 1 delim char : replace all '=' with '|'
       in the string
       var parts = x.split('|'); // JavaScript version: non-
       capital "s" !!!
       ImageSeries_myCloud = parts[1];
       ImageSeries_myTenantStorage = parts[3];
       ImageSeries_myTenantCompute = parts[5];
       ImageSeries_myProcedure = parts[7];
       ImageSeries_myAppHost = parts[9];
       ImageSeries_myConfigHost = parts[11];
       ImageSeries_myComputeHost = parts[13];
       ImageSeries_myStorageHost = parts[15];
       ImageSeries_myStorageHostVolumeSeries2d = parts[17];
       ImageSeries_myStorageHostVolumeSeries2dAnz =
       parts[19];
       set_ModeButton_Visibility("Visible"); // ModeBut-
       ton.Visibility = Visibility.Visible;
       _config_content_type = "volume";
       mContent = "volume";
       _imaging_mode = "3D"; // current will be 3d
       now
       set_ModeButton_Content("2D"); // ModeButton.Content =
       "2D";
       _loop_mode = "3DCine"; // current will be 3d
       eine
       set_3D_Image_Visibility(true); // msi1.Opacity = 100;
       set_2D_Image_Visibility(false); // msi.Opacity = 0;
       _Menue3dPressed = true;
   _imaging_mode = "3D";
   GUI_setDefaultBars();
      setInstanceInfo (myInstanceInfo); // new 30.1.2013
      Volume_fromListBox(mState, mGuid, mStorageType, mStor-
      ageFormat, mdrive, mnameUC, mDZMHDDataRelPath, mServer-
      BaseUri, mDZMetaDataRelPath, mDZInstDataRelPath, mContent,
      true);
   }
   else
   {
       mode = "2D";
       set_ListTitle_Text(" 2D Viewing");
       set_ModeButton_Visibility("Collapsed"); // ModeBut-
       ton.Visibility = Visibility.Collapsed;
       _imaging_mode = "2D"; // current will be 2d
       now
       set_ModeButton_Content("3D"); // ModeButton.Content =
       "3D";
       if (mContent == "image series")
           _loop_mode = "2DCine"; // current will
           be eine
       else
           _loop_mode = "Review"; // current will
           be review
       set_3D_Image_Visibility(false); // msi1.Opacity = 0;
       set_2D_Image_Visibility(true); // msi.Opacity = 100;
       _Menue3dPressed = false;
       _MSI_loading = true;
       if (mContent == "Image" && mDZMetaDataRelPath ==
       "jpeg" && mDZMHDDataRelPath == "None")
       {
         var x = mServerBaseUri.replace(/=/g, "|"); // Ja-
         vaScript supports only 1 delim char : replace all '=' with
         '|' in the string
         var parts = x.split('|'); // JavaScript version:
         non-capital "s" !!!
         //
         ImageSeries_myCloud = parts[1];
         ImageSeries_myTenantStorage = parts[3];
         ImageSeries_myTenantCompute = parts[5];
         ImageSeries_myProcedure = parts[7];
         ImageSeries_myAppHost = parts[9];
         ImageSeries_myConfigHost = parts[11];
         ImageSeries_myComputeHost = parts[13];
         ImageSeries_myStorageHost = parts[15];
         ImageSeries_myStorageHostVolumeSeries2d =
         parts[17];
         ImageSeries_myStorageHostVolumeSeries2dAnz =
         parts[19];
         var Cloud_imageJpegPath = mServerBaseUri +
         mDZInstDataRelPath;
        _imaging_mode = "2D";
         GUI_setDefaultBars();
         tweetUrl = Cloud_imageJpegPath;
         OnNavigatedToCanvas(tweetUrl); }
       else
       {
               if (mContent == "image series")
               {
              var x = mServerBaseUri.replace(/=/g, "|");
              var parts = x.split('|'); // JavaScript ver-
              sion: non-capital "s" !!!
              ImageSeries_myCloud = parts[1];
              ImageSeries_myTenantStorage = parts[3];
              ImageSeries_myTenantCompute = parts[5];
              ImageSeries_myProcedure = parts[7];
              ImageSeries_myAppHost = parts[9];
              ImageSeries_myConfigHost = parts[11];
              ImageSeries_myComputeHost = parts[13];
              ImageSeries_myStorageHost = parts[15];
              ImageSeries_myStorageHostVolumeSeries2d =
              parts[17];
              ImageSeries_myStorageHostVolumeSeries2dAnz =
              parts[19];
              ServerBaseUri = ImageSeries_myStorageHost;
               }
               else
               {
                   ServerBaseUri = mServerBaseUri;
               }
        _imaging_mode = "2D";
         GUI_setDefaultBars();
         var Cloud_imageDzcPath = ServerBaseUri + mDZInstDa-
         taRelPath;
         tweetUrl = Cloud_imageDzcPath;
         OnNavigatedToCanvas(tweetUrl) ;
         return; } } }
```

### Bezugszeichenliste

- 1: System
- 2,2': (medizinische) Einrichtung
- 3: Modalität
- 4: Computertomograph
- 5: C-Bogengerät
- 6: Magnetresonanztomograph
- 7: (Steuer- und Auswerte-)Rechner
- 8: Device
- 9: Personal-Computer
- 10: Bildschirm
- 11: Tablet-Computer
- 12: Smartphone
- 13: Public Cloud
- 14: (Datenübertragungs-)Netz
- 15: Intranet
- 16: Internet
- 17: Firewall
- 18,18': Subscription
- 19: Cloud Storage
- 20: App Store
- 21: Cloud-Compute-Service
- 22: (Patient-)Hub
- 23: (Worklist-)Hub
- 24: (Context-)Hub
- 25: (Image-)Hub
- 26: Table-Storage
- 27: Tweet
- 28: Blob-Storage
- 29: URI
- 31: Applikation
- 32: Applikation
- 33: Applikation
- 34: Applikation
- 35: Feed
- 36: GUI-Seite
- 37: (graphische) Benutzeroberfläche
- 40: Kopfzeile
- 41: Überschriftenfeld
- 42: Anzeigefeld
- 43: Befehlsleiste
- 44: Schaltfläche
- 45: Schaltfläche
- 46: Rahmen
- 50: Rahmenschicht
- 51: Rahmenkomponente
- 52: View-Schicht
- 53: Komponente
- 54: Komponente
- 55: ViewModel-Schicht
- 56: Komponente
- 57: Model-Schicht
- 58: Komponente
- 59: Treiberschicht
- 60: Treiberkomponente
- 61: Treiberkomponente

- P: Patientendatensatz
- W: Aufgabe
- C: Kontextdatensatz
- B: Bilddatensatz
- V: Ansicht
- Q: Suchanfrage
- R: Anfrage
- K: Keyimage

## Patentansprüche

1. System zur Verwaltung und Bearbeitung von Daten einer medizinischen Einrichtung (2,2'), mit einem Cloud-Storage (19) einer Public Cloud (13) und mindestens einer in dem Cloud-Storage (19) vorgehaltenen Viewing-Applikation (34) zum Ablauf auf einem Nutzergerät (8,9,11,12), wobei die Viewing-Applikation (34) zum Anzeigen von in dem Cloud Storage (19) vorgehaltenen medizinischen Bilddatensätzen (B) eingerichtet ist, wobei die Viewing-Applikation (34) hinsichtlich ihrer Komponenten-Architektur folgende Schichten umfasst:
- eine View-Schicht (52), umfassend mindestens eine Komponente (53,54), die den graphischen Gehalt einer GUI-Seite (36) zur Anzeige von 2D-Bilddaten oder 3D-Bilddaten definiert,
- eine ViewModel-Schicht (55), umfassend eine Komponente (56), die für die oder jede Komponente (53,54) der View-Schicht (52) Funktionen für die Steuerelemente der dort definierten GUI-Seite (36) zur Anzeige und Bearbeitung der Bilddaten definiert,
- eine Model-Schicht (57), umfassend eine Komponente (58), die Variablen für die anzuzeigenden Bilddaten definiert, sowie
- eine Treiberschicht (59), umfassend
- eine Komponente (60), die dazu eingerichtet ist, 2D-Bilddatensätze (B) zu laden und für die Anzeige auf einem Bildschirm aufzubereiten,
- optional zuladbar oder aktivierbar eine weitere Komponente (60), die dazu eingerichtet ist, 3D-Bilddatensätze (B) oder daraus abgeleiteten zweidimensionalen Ansichten (V) zu laden und für die Anzeige auf einem Bildschirm aufzubereiten,
wobei die Komponente (56) der ViewModel-Schicht (55) generisch ist in dem Sinne, dass sie ein gemeinsames Set von Funktionen für die Anzeige und Bearbeitung von 2D-Bilddaten und 3D-Bilddaten definiert,
wobei die anzuzeigenden Bilddatensätze (B) in dem Cloud-Storage (19) der Public Cloud (13) derart vorgehalten sind, dass auf jeden Bilddatensatz (B) bei Vorliegen einer entsprechenden Cloud-Subscription (18) über einen zugehörigen URL (29) zugegriffen werden kann, und wobei die Viewing-Applikation (34) aus dem Cloud-Storage (19) der Public Cloud (13) bei Vorliegen einer entsprechenden Cloud-Subscription (18) über einen zugehörigen URL heruntergeladen werden kann, wobei der URL eines anzuzeigenden Bilddatensatzes (B) als Argument spezifizierbar ist.

2. System (1) nach Anspruch 1,
mit einer der View-Schicht (52) übergeordneten Rahmenschicht (50), umfassend eine Komponente (51), die einen generischen Ablauf-Rahmen (46) für die oder jede Komponente (53,54) der View-Schicht (52) zur Verfügung stellt.

3. System (1) nach Anspruch 1 oder 2,
wobei jede Treiberkomponente (60,61) dazu eingerichtet ist, Anfragen von Komponenten der übergeordneten Schichten (55) asynchron und parallel zu bearbeiten, wobei die oder jede Treiberkomponente (60,61) nur durch Rückgabe der aufgrund der Anfrage geladenen und/oder aufbereiteten Bilddaten antwortet.

4. System (1) nach einem der Ansprüche 1 bis 3,
wobei die Treiberkomponente (60) zum Laden von 2D-Bilddaten dazu eingerichtet ist, Bilddatensätze (B) unmittelbar ohne Inanspruchnahme von Serverleistung in Form von Cloud-Compute-Diensten aus einem vorbestimmten Datenspeicher zu laden.

5. System (1) nach Anspruch 4,
wobei die Treiberkomponente (60) zum Laden von 2D-Bilddaten dazu eingerichtet ist, die Bilddaten durch unmittelbaren Zugriff auf einen Cloud-Storage (19) einer Public Cloud (13) zu laden.

6. System (1) nach einem der Ansprüche 1 bis 5,
wobei die Treiberkomponente (61) zum Laden von 3D-Bilddaten oder daraus abgeleiteten zweidimensionalen Ansichten (V) dazu eingerichtet ist, in einem Online-Betriebsmodus zur Erzeugung der zweidimensionalen Ansichten (V) aus einem anzuzeigenden 3D-Bilddatensatz (B) auf Cloud-Computer-Dienste (21) zugreifen.

7. System (1) nach Anspruch 6,
wobei die Treiberkomponente (61) zum Laden von 3D-Bilddaten oder daraus abgeleiteten zweidimensionalen Ansichten (V) darüber hinaus dazu eingerichtet ist, in einem Offline-Modus die anzuzeigenden Daten direkt ohne Inanspruchnahme eines zwischengeschalteten Servers aus einem Datenspeicher (19) zu laden und auf dem Nutzergerät (8) die zweidimensionalen Ansichten (V) der dreidimensionalen Bildinformation zur Darstellung auf einem Bildschirm des Nutzergeräts (8) ableiten.

8. System (1) nach Anspruch 1,
wobei die Viewing-Applikation (34) zur Anzeige eines Bilddatensatzes (B) unter Angabe des URL (29) dieses Bilddatensatzes (B) als Argument aufrufbar ist.

## Claims

1. System for management and processing of data of a medical facility (2, 2'), with cloud storage (19) of a public cloud (13) and at least one viewing application (34) held in the cloud storage (19) for execution on a user device (8, 9, 11, 12), wherein the viewing application (34) is configured for displaying medical image datasets (B) held in the cloud storage (19), wherein the viewing application (34) comprises the following layers in respect of its component architecture:
- a View layer (52), comprising at least one component (53, 54) that defines the graphical content of a GUI page (36) for display of 2D image data or 3D image data,
- a ViewModel layer (55), comprising one component (56), which, for the or for each component (53, 54) of the View layer (52), defines functions for the control elements of the GUI page (36) defined there for display and processing of the image data,
- a Model layer (57), comprising one component (58), which defines variables for the image data to be displayed, and also
- a Driver layer (59), comprising
- a component (60), which is configured to load 2D datasets (B) and edit them for display on a screen,
- a further component (60), able to loaded or activated as an option, which is configured to load 3D image datasets (B) or two-dimensional views (V) derived therefrom and edit them for display on a screen, wherein the component (56) of the ViewModel layer (55) is generic in the sense that it defines a common set of functions for the display and processing of 2D image data and 3D image data,
wherein the image datasets (B) to be displayed are held in the cloud storage (19) of the public cloud (13) in such a way that there can be access to each image dataset (B) via an associated URL (29), if a corresponding cloud subscription (18) is available, and
wherein the viewing application (34) can be downloaded via an associated URL from the cloud storage (19) of the public cloud (13), if a corresponding cloud subscription (18) is available, wherein the URL of an image dataset (B) to be displayed can be specified as an argument.

2. System (1) according to claim 1,
with a Frame layer (50) superordinate to the View layer (52), comprising a component (51), which makes available a generic execution frame (46) for the component or for each component (53, 54) of the View layer (52).

3. System (1) according to claim 1 or 2,
wherein each driver component (60, 61) is configured to process requests from components of the superordinate layers (55) asynchronously and in parallel, wherein the driver component or each driver component (60, 61) only responds by returning the image data loaded and/or edited because of the request.

4. System (1) according to one of claims 1 to 3,
wherein the driver component (60) for loading 2D image data is configured to load image datasets (B) from a predetermined data memory directly without making use of server power in the form of cloud computing services.

5. System (1) according to claim 4,
wherein the driver component (60) for loading 2D image data is configured to load the image data by direct access to cloud storage (19) of a public cloud (13).

6. System (1) according to one of claims 1 to 5,
wherein the driver component (61) for loading 3D image data or two-dimensional views (V) derived therefrom is configured to access cloud computer services (21) in an online operating mode to create the two-dimensional views (V) from a 3D image dataset (B) to be displayed.

7. System according to claim 6,
wherein the driver component (61) for loading 3D image data or two-dimensional views (V) derived therefrom is additionally configured, in an offline mode, to load the data to be displayed directly from a data memory (19) without making use of an intermediate server and to derive on the user device (8) the two-dimensional views (V) of the three-dimensional image information for display on a screen of the user device (8).

8. System (1) according to claim 1,
wherein the viewing application (34) for displaying an image dataset (B) is able to be called by specifying the URL (29) of this image dataset (B) as an argument.

## Revendications

1. Système de gestion et de traitement de données d'un dispositif (2, 2) médical, comprenant une mémoire (19) de nuage d'un nuage (13) public et au moins une application (34) de vision mise en avant dans la mémoire (19) de nuage pour se dérouler sur un appareil (8, 9, 11, 12) d'utilisateur, l'application (34) de vision étant conçue pour l'affichage d'ensembles (B) de données d'image médicale maintenus en avant dans la mémoire (19) de nuage, dans lequel l'application (34) de vision comprend, en ce qui concerne son architecture de composants, les couches suivantes :
- une couche (52) de vue, comprenant au moins un composant (53, 54), qui définit la teneur graphique d'une page (36) GUI pour l'affichage de données d'image en 2D ou de données d'image en 3D,
- une couche (55) de modèle de vue, comprenant un composant (56), qui définit pour l'affichage et le traitement des données d'image, pour le ou chaque composant (53, 54) de la couche (52) de vue, des fonctions pour les éléments de commande de la page (36) GUI, qui y est définie,
- une couche (57) de modèle, comprenant un composant (58), qui définit des variables pour les données d'image à afficher, ainsi que
- une couche (59) d'attaque, comprenant
- un composant (60), qui est conçu pour charger des ensembles (B) de données d'image en 2D et pour les préparer pour l'affichage sur un écran,
- un autre composant (60), pouvant être chargé ou activé facultativement, qui est conçu pour charger des ensembles (B) de données d'image en 3D ou des vues (V) en deux dimensions, qui s'en déduisent, et pour les préparer pour l'affichage sur un écran,
dans lequel le composant (56) de la couche (55) de modèle de vue est générique en ce sens, qu'il définit un jeu commun de fonctions, pour l'affichage et le traitement de données d'image en 2D et de données d'image en 3D,
dans lequel les ensembles (B) de données d'image à afficher dans la mémoire (19) de nuage du nuage (13) public sont maintenus en avant, de manière à ce qu'on puisse accéder à chaque ensemble (B) de données d'image, en présence d'une subscription (18) de nuage correspondante, et dans lequel l'application (34) de vison peut être chargée en-dessous à partir de la mémoire (19) de nuage du nuage (13) public, en présence d'une subscription (18) de nuage correspondante, par un URL (29) associé, 1'URL d'un ensemble (B) de données d'image à afficher pouvant être spécifié comme argument.

2. Système (1) suivant la revendication 1,
comprenant une couche (50) de cadre supérieure hiérarchiquement à la couche (52) de vue et comprenant un composant (51), qui met à disposition un cadre (46) de déroulement générique pour le ou pour chaque composant (53, 54) de la couche (52) de vue.

3. Système (1) suivant la revendication 1 ou 2,
dans lequel chaque composant (60, 61) d'attaque est conçu pour traiter, de manière asynchrone et en parallèle, des demandes de composants des couches (55) supérieures hiérarchiquement, dans lequel le ou chaque composant (60, 61) d'attaque ne répond que par restitution des données d'image chargées en raison de la demande et/ou préparées.

4. Système (1) suivant l'une des revendications 1 à 3,
dans lequel le composant (60) d'attaque est conçu pour le chargement de données d'image en 2D pour charger, dans une mémoire de données définie à l'avance, des ensembles (B) de données d'image directement, sans mise en jeu de puissance de serveur sous la forme de services d'ordinateur de nuage.

5. Système (1) suivant la revendication 4,
dans lequel le composant (60) d'attaque est conçu pour la charge de données d'image en 2D pour charger les données d'image par accès direct à une mémoire (19) de nuage d'un nuage (13) public.

6. Système (1) suivant l'une des revendications 1 à 5,
dans lequel le composant (61) d'attaque est conçu pour la charge de données d'image en 3D ou de vues (V) en deux dimensions qui s'en déduisent, pour accéder à des services (21) d'ordinateur de nuage, dans un mode de fonctionnement en ligne pour la production des vues (V) en deux dimensions à partir d'un ensemble (B) de données d'image en 3D à afficher.

7. Système (1) suivant la revendication 6,
dans lequel le composant (61) d'attaque est conçu en outre pour le chargement de données d'image en 3D ou de vues (V) en deux dimensions qui s'en déduisent, pour charger, à partir d'une mémoire (19) de données dans un mode hors ligne, les données affichées directement sans mise en jeu d'un serveur intermédiaire et pour dériver sur l'appareil (8) d'utilisateur les vues (V) en deux dimensions de l'information d'image en trois dimensions en vue de la représentation sur un écran de l'appareil (8) d'utilisateur.

8. Système (1) suivant la revendication 1,
dans lequel l'application (34) de vision peut être appelé comme argument pour l'affichage d'un ensemble (B) de données d'image en indiquant le URL (29) de cet ensemble (B) de données d'image.
